# EUROPEAN PATENT SPECIFICATION

(11) **EP 1 871 311 B1**
(45) Date of publication and mention of the grant of the patent: **26.11.2014**
(21) Application number: 06751015.6
(22) Date of filing: 20.04.2006
(51) Int. Cl.: A61F 2/95

(54) **JOINT FOR MEDICAL DEVICE DELIVERY SYSTEM**
GELENK FÜR SYSTEM ZUR ZUFÜHRUNG VON MEDIZINISCHEN GERÄTEN
JOINT SYSTEME D'IMPLANTATION DE DISPOSITIF MEDICAL

(30) Priority: 20.04.2005 US 673199 P
(43) Date of publication of application: 02.01.2008
(73) Proprietor: Cook Medical Technologies LLC, Bloomington, IN 47404 (US)
(72) Inventor: PAL, Dharmendra, Wilmington, Massachusetts 01887 (US); MELSHEIMER, Jeffrey, S., Springville, Indiana 47462 (US); RICHARDS, Kathryn, E., Hershey, PA 17033 (US)
(74) Representative: Mathys & Squire LLP
(86) International application number: PCT/US2006/015150
(87) International publication number: WO 2006/113913

(56) References cited:
- EP-A- 1 095 634
- WO-A-00/24450

## Description

### Technical Field

The present invention relates generally to a joint assembly for operatively coupling an inner compression member and an inner guide channel member used for medical device delivery systems that deploy an implantable prosthesis such as self-expanding, balloon expandable, or non-expanding stents, prosthetic valve devices, and other implantable articles (individually and collectively, "stent," "stents," "implantable prosthesis," and "implantable prostheses") at a selected location inside a patient's body.

### Background of the Invention

This invention relates generally to medical device delivery systems, and in particular, to a joint assembly operatively coupling an inner compression member and an inner compression member for use in medical devices that employ a catheter. These medical device delivery systems have a host of uses, including, for example, the deployment of a self-expanding implantable prosthesis at selected locations inside a patient's body. The invention may also be used, however, with a balloon expandable and non-expanding implantable prosthesis. In addition to being used with a rapid insertion delivery system, the invention may be used in an "over-the-wire" delivery system, so both systems will be described below.

By way of background, stents are configured to be implanted into body vessels having a passageway in order to reinforce, support, repair, or otherwise enhance the performance of the passageway. The term "passageway" is understood to be any lumen, channel, flow passage, duct, chamber, opening, bore, orifice, or cavity for the conveyance, regulation, flow, or movement of bodily fluids and/or gases of an animal. As an example, stents have been used in the passageways of an aorta, artery, bile duct, blood vessel, bronchiole, capillary, esophagus, fallopian tube, heart, intestine, trachea, ureter, urethra, vein, and other locations in a body (collectively, "vessel") to name a few.

One type of stent is self-expanding. For a self-expanding stent, the stent is resiliently compressed into a collapsed first, smaller diameter, carried by the delivery system, and due to its construction and material properties, the stent expands to its second, larger diameter upon deployment. In its expanded configuration, the stent exhibits sufficient stiffness so that it will remain substantially expanded and exert a radially outward force in the vessel passageway on an interior surface of the vessel. One particularly useful self-expanding stent is the Z-stent, introduced by Cook Incorporated, due to its ease of manufacturing, high radial force, and self-expanding properties. Examples of the Z-stent are found in United States Patent Nos. 4,580,568; 5,035,706; 5,282,824; 5,507,771; and 5,720,776. The Zilver stent, introduced by Cook Incorporated, is another particularly useful self-expanding stent due to its nitinol platform and use of the Z-stent design properties. Examples of the Zilver stent are found in United States Patent Nos. 6,743,252 and 6,299,635.

Many delivery systems employ a tubular catheter, sheath, or other introducer (individually and collectively, "catheter") having first and second ends and comprising a lumen for receiving the wire guide. Optionally, these delivery systems may fit through a working channel within an endoscope or an external accessory channel device used with an endoscope.

Generally stated, these delivery systems may fall within two categories. The first category of delivery systems to have been used, and consequently the first to be discussed below, is commonly referred to as an "over-the-wire" catheter system. The other category of delivery systems is sometimes referred to as a "rapid exchange" catheter system. In either system, a wire guide is used to position the delivery system within a vessel passageway. The typical wire guide has proximal and distal ends. A physician inserts the distal end into the vessel passageway, advances, and maneuvers the wire guide until the distal end reaches its desired position within the vessel passageway.

In the "over-the-wire" catheter delivery system, a physician places the catheter over the wire guide, with the wire guide being received into a lumen that extends substantially through the entire length of the catheter. In this over-the-wire type of delivery system, the wire guide may be back-loaded or front-loaded into the catheter. In front-loading an over-the-wire catheter delivery system, the physician inserts the distal end of the wire guide into the catheter's lumen at or near the catheter's proximal end. In back-loading an over-the-wire catheter delivery system, the physician inserts a distal portion of the catheter over the proximal end of the wire guide. The back-loading technique is more common when the physician has already placed the wire guide into the patient, which is typically the case today. In either case of back-loading or front-loading an over-the-wire catheter delivery system, the proximal and distal portions of the catheter will generally envelop the length of the wire guide that lies between the catheter first and second ends. While the wire guide is held stationary, the physician may maneuver the catheter through the vessel passageway to a target site at which the physician is performing or intends to perform a treatment, diagnostic, or other medical procedure.

Unlike the over-the-wire system where the wire guide lies within the catheter lumen and extends substantially the entire length of the catheter, in a "rapid insertion" catheter delivery system described in application serial number 60/673,199, the wire guide occupies a catheter lumen extending only through a distal segment of the catheter. The so-called rapid insertion system comprises a system proximal end, an elongate flexible middle section and a system distal end that is generally tubular.

The system distal end, in general, comprises an inner guide channel member sized to fit within an outer guide channel member that is substantially axially slideable relative to the inner guide channel member. The outer guide channel member and inner guide channel member further have entry and exit ports defining channels configured to receive a wire guide. A port includes any structure that functions as an entry or exit aperture, cutout, gap, hole, opening, orifice, passage, passageway, port, or portal, while a guide channel is understood to be any aperture, bore, cavity, chamber, channel, duct, flow passage, lumen, opening, orifice, or passageway that facilitates the conveyance, evacuation, flow, movement, passage, regulation, or ventilation of fluids, gases, or a diagnostic, monitoring, scope, other instrument, or more particularly a catheter or wire guide.

A wire guide may extend from the outer and inner member entry ports, through the outer and inner member guide channels, and exit the distal end at or near a breech position opening located at or near a transition region where the guide channels and exit ports are approximately aligned relatively coaxially to facilitate a smooth transition of the wire guide. Furthermore, the outer guide channel member has a slightly stepped profile, whereby the outer guide channel member comprises a first outer diameter and a second smaller outer diameter proximal to the first outer diameter and located at or near the transition region.

The system distal end also has a self-expanding deployment device mounting region (e.g., a stent mounting region) positioned intermediate the inner guide channel member entry and exit ports for releasably securing a stent. At the stent mounting region, a stent is releasably positioned axially intermediate distal and proximal restraint markers and sandwiched transversely (i.e., compressed) between the outside surface of the inner guide channel member and the inside surface of an outer guide channel member.

Turning to the system proximal end of the rapid insertion delivery system, the proximal end, in general, comprises a handle portion. The handle portion has a handle that the physician grips and a pusher stylet that passes through the handle. The pusher stylet is in communication with-directly or indirectly through intervening parts-the inner guide channel member at the distal end. Meanwhile, the handle is in communication with-directly or indirectly through intervening parts-the outer guide channel member at the distal end. Holding the pusher stylet relatively stationary (while, for example, actuating the handle) keeps the stent mounting region of the inner guide channel member properly positioned at the desired deployment site. At the same time, proximally retracting the handle results in a corresponding proximal movement of the outer guide channel member relative to the inner guide channel member to thereby expose and, ultimately, deploy the self-expanding stent from the stent mounting region. At times, a physician may need to deploy a second self-expanding stent by withdrawing the system from the proximal end of the wire guide. The physician may then reload the catheter with additional stents, and if that is not an option the physician may load another stent delivery system with an additional stent, onto the wire guide. Also, the physician may withdraw the stent delivery system altogether and replace the delivery system with a catheter or different medical device intended to be loaded onto the wire guide.

The delivery system in the rapid insertion delivery system further comprises an elongate flexible middle section delivery device extending intermediate the system proximal end and the system distal end. The middle section delivery device comprises an outer sheath and an inner compression member having first and second ends associated with the system distal end and system proximal end, respectively. An exemplary rapid exchange stent delivery catheter system is shown in EP-A-1095634.

More particularly, the outer sheath first end may be coterminous with or, if separate from, may be associated with (e.g., joined or connected directly or indirectly) the distal end outer guide channel member at or near the transition region, while the outer sheath second end is associated with the handle at the system proximal end. The inner compression member first end is associated with the distal end inner guide channel member at or near the transition region, while the inner compression member second end is associated with the pusher stylet at the proximal end. Therefore, the outer guide channel member of the distal end may move axially (as described above) and independently relative to an approximately stationary inner guide channel member of the system distal end and, thereby, deploy the stent.

A challenge in designing a delivery system in the rapid exchange delivery system is that the handle portion is the only interface through which the operator can control the working parts, and the inner compression member for use with the system typically forms the only connection to the inner guide channel member sometimes 50.0 centimeters and often as much or more than 125 centimeters distal to the handle portion. Before the "rapid insertion" catheter delivery system described in application serial number 60/673, 199, the ways of associating the inner compression member first end to the inner guide channel member have typically been to use a mechanical lap joint.

A lap joint describes a technique for joining two pieces of material by overlapping them. A lap may be a full lap or half lap. In a full lap, no material is removed from either of the members to be joined, resulting in a joint which is the combined thickness of the two members. Unfortunately, the full lap joint increases the profile of the distal end. In a half lap joint, material is removed from each of the members so that the resulting joint is the thickness of the thickest member. Thus, half lap joints require that each member be cut to a complementary surface, which is an arduous task in view of the already small thickness of the inner compression member and thin walls of the inner guide channel member.

The present invention solves these and other problems by forming the inner compression member distal mating end to modify it from a round configuration to a low profile.

Another problem with the mechanical lap joint is that it requires some form of mechanical fastener to be effective. A fastener is a hardware device that mechanically joins or affixes two or more members together. Like a band clamp and the like, the fastener typically disposes about the two members that have joined, and therefore requires a friction fit to hold the two members in a non-displaceable arrangement. Moreover, the fastener adds to the profile of the two members and the fastener, and thereby confounds the goal of minimizing the profile of the distal end.

The present invention solves these and other problems by providing a securing body having a receiving cavity for the inner guide channel member and a base section for the contoured inner compression member distal mating end.

Another drawback to the fastener in a mechanical lap joint connection is the propensity to lose the friction fit between the members. Accordingly, a glued joint is often employed as an alternative to a mechanical lap joint. While glue, adhesives, and the like (collectively, "glue") offer advantages over a mechanical joint, one must choose the right glue to join dissimilar materials. In any event, lap joints and glued joints may vary in strength and integrity depending on the type of materials being joined and whether the materials have incongruous mating surfaces. In addition, the point attachments that are typically formed by these joints could cause joint failure due to inadequate stress distribution, and may detach when a torque-load is applied.

The present invention solves these and other problems by joining the inner guide channel member within the receiving cavity of the securing body while operatively coupling the inner compression member to the securing body base section with welding, fusing, soldering, brazing, cementing, and the like.

Therefore, it would be desirable to have an internal joint for a medical device delivery system for self-expanding devices such as stents, prosthetic valve devices, and other implantable articles inside a patient's body as taught herein.

### Summary of the Invention

The present invention relates a joint assembly for medical device delivery systems. In one embodiment, an inner guide channel member has first and second ends, the second end having an outer diameter and outer surface. An elongate inner compression member has a proximal end and distal mating end with a contoured engaging surface. A securing body includes an inner securing section with a receiving cavity for disposing about and operatively coupling to the inner guide channel member second end outer surface, and a base securing section with an outer engaging surface disposed against the inner compression member distal mating end engaging surface, the base section and distal mating end being operatively coupled.

In another embodiment, the invention provides a medical device delivery system for deploying an implantable prosthesis at a selected location inside a patient's body. The delivery system includes a cannulated securing body with an inner securing section and a base securing section. An elongate inner compression member has a distal mating end operatively coupled to the cannulated securing body base securing section. An inner guide channel member has a first end operatively coupled to an atraumatic tip and a second end operatively coupled to the cannulated securing body inner securing section. A stent platform and a restraining ring thereto are disposed circumferentially intermediate the inner guide channel member first and second ends.

In yet another embodiment, the present invention provides a method of making an internal joint for use in a medical device delivery system. An inner guide channel member is provided with first and second ends defining a guide channel, the second end having an outer diameter and an outer surface. A securing body is provided with an inner securing section having a receiving cavity sized to receive the inner guide channel member second end outer diameter, and having a base securing section having an outer engaging surface. An elongate inner compression member is provided with a distal mating end, which is formed to an engaging surface having a contoured configuration complementary to the base securing section outer engaging surface and operatively coupled against the base securing section outer engaging surface. The inner guide channel member second end is inserted within the securing body inner securing section receiving cavity and operatively coupled to the inner securing section.

### Brief Description of the Drawing

Figure 1 is a schematic view, broken away, of a medical device system.
Figure 2 is an exploded side view, broken away, of a proximal end of a medical device.
Figure 2A shows longitudinally sectioned exploded side views of a handle first connector and a handle second connector.
Figure 2B shows a longitudinally sectioned side view of operatively coupled first and second connectors according to Figures 2A.
Figure 2C shows a longitudinally sectioned side view of a handle first connector and a handle second connector according to Figure 2B operatively coupling a strain relief member and/or an outer sheath according to one embodiment of the invention.
Figure 3 is a longitudinally sectioned view along a partial length of an embodiment of a catheter used for an outer sheath of a middle section and/or for an outer guide channel member of a distal end of a medical device according to the invention.
Figure 4 is a longitudinally sectioned view, broken away, of a distal end of medical device delivery system.
Figures 4A and 4B schematically represent cross sectional views of melt bonding according to one embodiment of the invention, where Figure 4A shows components before melt bonding while Figure 4B shows the components after melt bonding.
Figure 5 is a longitudinally sectioned view, broken away, of an alternative embodiment of a distal end of a medical device delivery system.
Figure 6 is a longitudinally sectioned view of an embodiment of a distal end according to the invention, shown having a portion of a wire guide.
Figure 7 is a longitudinally sectioned view, broken away, of another embodiment of a distal end of a medical device delivery system.
Figures 7A, 7B, and 7C show cross sectional views of Figure 7 taken along the lines 7A-7A, 7B-7B, and 7C-7C, respectively.
Figures 8A and 8B show perspective schematic views of a securing body according to one embodiment of the invention.
Figures 9A and 9B show perspective schematic views of a securing body according to an alternative embodiment of the invention.
Figure 10 shows a schematic side view of an inner compression member according to an embodiment of the invention.
Figures 10A, 10B, 10C, 10D, 10E, 10F, 10G, and 10H show cross sectional views of Figure 10 taken along the lines 10A-10A, 10B-10B, 10C-10C, 10D-10D, 10E-10E, 10F-10F, 10G-10G, 10H-10H, respectively.
Figures 10I, 10J, 10K, 10L, and 10M show perspective schematic views of alternative embodiments of an inner compression member distal mating end according to the invention.
Figure 11 shows a longitudinally sectioned schematic view of an inner compression member distal mating end operatively coupled to a base section of a securing body according to embodiments of the invention.
Figures 11A and 11B show cross sectional views of Figure 11 taken along the lines 11A-11A and 11 B-11 B, respectively.
Figure 12 shows a longitudinally sectioned view of an inner guide channel member assembly according to one embodiment of the invention.
Figures 12A, 12B, and 12C show cross sectional views of Figure 12 taken along the lines 12A-12A, 12B-12B, and 12C-12C, respectively.
Figure 13 shows a perspective schematic view of a shrink tubing according to one embodiment of the invention.
Figure 14 shows a longitudinally sectioned view of an inner guide channel member assembly having a securing body that operatively couples to the inner guide channel member and the inner compression member according to one embodiment of the invention.
Figures 14A shows a cross sectional view of Figure 14 taken along the line 14A-14A.
Figure 15 is a block diagram illustrating a method of manufacturing an internal joint for use in a medical device delivery system according to the present invention.

### Detailed Description

The present invention relates to medical devices, and in particular to an inner guide channel member assembly and to a joint assembly comprising a securing body operatively coupled to the inner guide channel member and an inner compression member joint assembly an inner compression member and also operatively coupling a low-profiled inner compression member for use in rapid insertion medical device delivery systems (e.g., "rapid insertion" catheters) configured for deploying expandable metallic, polymeric, and plastic devices or non-expanding metallic, polymeric, and plastic devices, which devices may include, by way of example and not by way of limitation, stents, prosthetic valve devices, and other implantable articles at selected locations inside a patient's body. For conciseness and ease of description of the embodiments of the invention, the term "stent" and its variations shall refer individually and collectively (without limiting the invention) to all self-expanding, balloon-expandable, or non-expanding devices used with the invention, such as stents, prosthetic valve devices, and other implantable articles inside a patient's body.

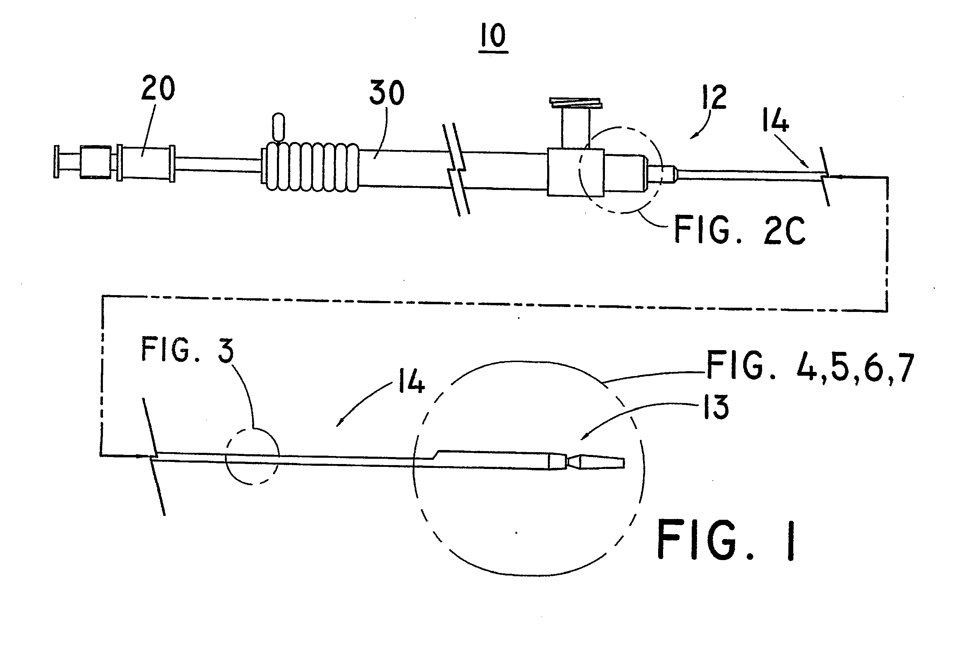

In Figure 1, an illustrative embodiment of a delivery system 10 having a host of uses, including for the rapid insertion of self-expanding stents, is provided. The delivery system 10 comprises a system proximal end 12, a middle section delivery device 14, and a system distal end 13 shown in a partially deploying position.

### SYSTEM PROXIMAL END 12

In the embodiment shown in Figure 1, the proximal end 12 remains outside of the patient's body. The proximal end 12 comprises a handle 30 and an optional pusher stylet 20.

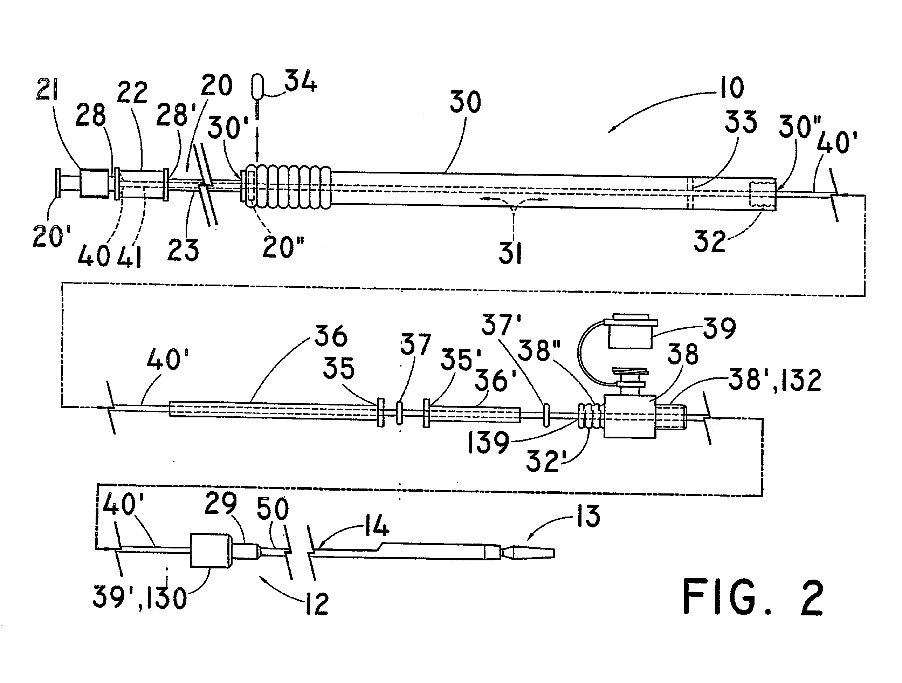

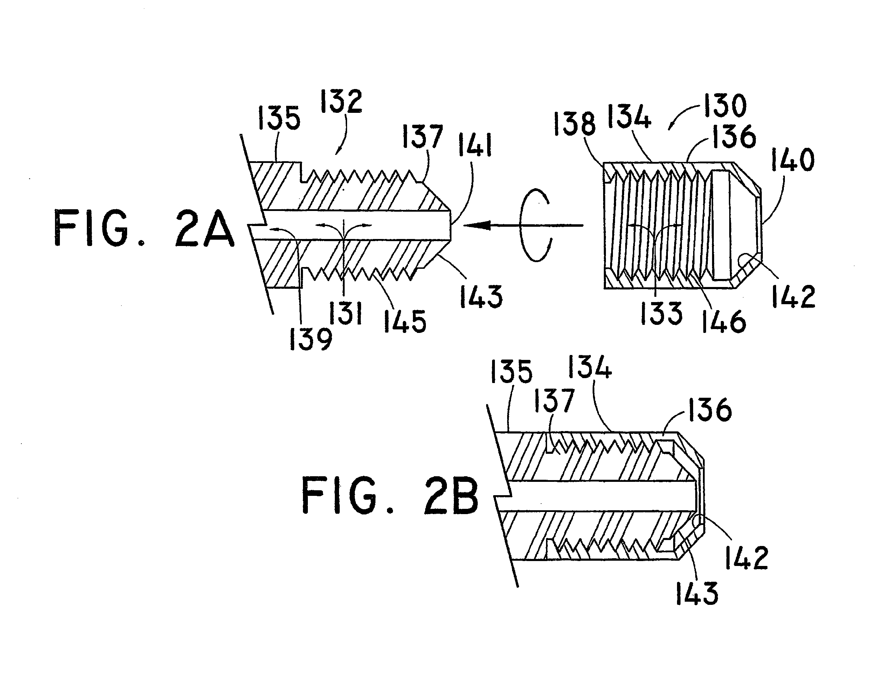

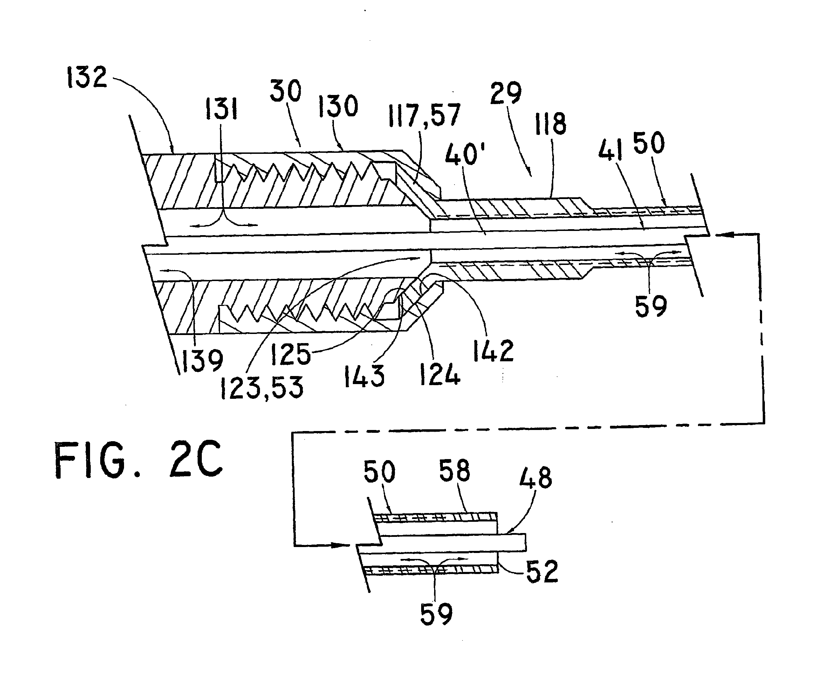

Figure 1 depicts a schematic representation of the handle 30 and the optional pusher stylet 20 shown more particularly in Figure 2. In general, a handle 30 retracts an outer guide channel member (discussed below) of the distal end 13 of the delivery system 10 to deploy a stent, as will be explained later. The handle 30 may comprise any mechanical, electromechanical, pneumatic, or hydraulic handle configured in communication with-directly or indirectly through intervening parts-the distal end's outer guide channel member. Communication would include, by way of illustration and not by way of limitation, a handle 30 that uses or is otherwise associated with, directly or indirectly, an elongated mechanical wire, rod, shaft, cable, sheath, pneumatic tube, or hydraulic pistons, cylinders and/or flow paths configured for moving the outer guide channel member proximally in order to deploy a stent.

Figure 2 provides a schematic view, broken away, of a delivery system 10 for rapid insertion of self-expanding stents, but could be used with other implantable prostheses described above. The delivery system 10 shown in Figure 2 is one embodiment of the proximal end 12, middle section delivery device 14, and distal end 13 shown in a partially deploying position. The middle section delivery device 14 extends distally from the proximal end 12, and a distal end 13 extends to a position that is distal the middle section delivery device 14. More particularly, Figure 2 shows an exploded view of the proximal end 12 of the delivery system 10 according to one embodiment of the invention, with an emphasis on the handle 30 and the optional stylet 20. Features of one embodiment of a handle 30 and pusher stylet 20 are discussed below.

The handle 30 comprises any tubular structure having a distal aperture 30" and a proximal aperture 30', the apertures defining a chamber 31 therebetween. In general, the handle 30 is a component, instrument, mechanism, tool, device, apparatus, or machine configured for directly or indirectly retracting an outer guide channel member (discussed below) of the distal end 13 of the device to expose and, ultimately, to deploy a stent self-expanding implantable prostheses such as stents, prosthetic valve devices, and other implantable articles (hereafter, "stent" or "stents") at a selected location inside a patient's body.

The handle 30 is axially slideable relative to an elongate (long) inner compression member 41 that comprises a proximal end 40 and a middle section 40'. As discussed more fully below, the inner compression member 41 helps to keep the stent from moving proximally with proximal movement of the handle 30, which handle movement causes the outer guide channel member to withdraw proximally over the stent in order to expose and thereby to deploy the stent. Thus, the inner compression member helps to "push" the stent or stent carrying inner guide channel member in order to counter the urge for the stent or stent carrying member to prolapse proximally with the withdrawing of the outer guide channel member. As will be understood, "pushing" on the inner compression member will keep the stent carrying inner guide channel member (and therefore the stent) from translating as a result of an outer sheath or outer guide channel member being pulled over the stent; thereby "pushing" holds the stent in place at the desired deployment site within the patient's body. In one embodiment, the handle 30 is a unidirectional handle that is axially slideable relative to the inner compression member 41 and/or the optional pusher stylet 20 in order to deploy a stent. In one embodiment, the inner compression member 41 is secured to a pusher stylet 20.

As shown in Figure 2, one embodiment of a pusher stylet 20 comprises a proximal end 20', a distal end 20", and a cannula 23 intermediate the proximal and distal ends 20', 20", respectively, and a receptacle 22. The cannula 23, as should be understood, comprises any suitable hollow plastic or metal tube. As a hollow tube, the cannula 23 optionally allows the inner compression member 41 to pass proximally through the cannula 23 and to the proximal end 20' so that the inner compression member proximal end 40 (such as a proximal end that is flared) may secure to a plug 21 that fits within the receptacle 22, wherein Figure 2 shows the proximal end 20', plug 21, and an optional securing material 28 are shown in an exploded view relative to the receptacle 22 into which they may be secured. Furthermore, the cannula 23 assists with keeping that portion of the inner compression member substantially straight.

The stylet 20 is optional, because in an alternative embodiment the physician may hold the inner compression member proximal end 40' directly in order to "push" (e.g., hold substantially stationary) the stent carrying inner guide channel member (and therefore the stent). This controls the stent carrying inner guide channel member and stent from translating as a result of an outer sheath or outer guide channel member being pulled over the stent, so that the stent remains at the desired deployment site within the patient's body. Alternatively, the stylet 20 is any stationary handle secured to the inner compression member 41 for achieving the "pushing" (e.g., hold substantially stationary) of the stent or stent carrying inner guide channel member while the outer sheath or outer guide channel member are moved proximally.

The stylet distal end 20" is housed within the handle chamber 31 and is flared or otherwise flanged sufficiently to be larger than the handle proximal aperture 30' so as not to pull out of the chamber 31. In one embodiment, the stylet distal end 20" is secured to the distal portion of the stylet cannula 23, while in another embodiment the stylet distal end 20" is formed integral with the distal portion of the stylet cannula 23. Consequently, the stylet distal end 20" functions as a proximal stop that prevents the stylet cannula 23 from backing all the way out the handle while being axially slideable within the handle chamber 31. Thus, the stylet 20 will not slide off the handle 30, if so desired. The stylet distal end 20" may also, in one embodiment, function as a distal stop against a restraint 33 formed in the handle chamber 31 intermediate the handle proximal and distal apertures 30', 30", respectively, where intermediate should be understood to be any position between, and not necessarily equidistant to, the handle apertures 30', 30". As a result of the stylet distal end 20", the handle 30 may slide axially the distance separating the handle restraint 33 and the stylet distal end 20", which has a maximum distance of when the stylet distal end 20" is abutting the handle proximal aperture 30'.

A threaded tapered plug 21 and threaded tapered receptacle 22 optionally secure the inner compression member proximal end 40. In one embodiment, the inner compression member proximal end 40 is flared. Securing material 28, such as glue, adhesives, resins, welding, soldering, brazing, chemical bonding materials or combinations thereof and the like (collectively and individually, "glue") may be used to keep the threaded tapered plug 21 from backing out of the threaded tapered receptacle 22. A portion of the cannula 23 and stylet distal end 20" are received within the handle chamber 31 distal to the handle proximal aperture 30' as previously explained.

By optionally placing the inner compression member proximal end 40 in mechanical communication with the plug 21 and receptacle 22, the gripping and "pushing" (e.g., hold substantially stationary) on the stylet 20 (e.g., the receptacle 22) thereby helps to keep the inner compression member 41 from moving away from the distal end 13 and, accordingly, counters the tendency for a stent or stent carrying member to move proximally during withdrawal of the outer guide channel member as will be explained below. Of course, the inner compression member may be secured elsewhere by the stylet 20, such as at or near the stylet distal end 20" or intermediate the stylet proximal and distal ends 20', 20", respectively, and the stylet distal end 20" may extend to a position at or near the distal end aperture 30" of the handle 30.

Figure 2 shows a middle section 40' that extends distally from the proximal end 40 of the inner compression member 41. In one embodiment, the middle section 40' passes through the handle 30 (and may pass through the cannula 23 and/or bushings housed within the handle chamber 31 or other portions of the proximal end 12). In one embodiment, the middle section 40' is elongate (at least 50.0 cm or longer as described below) and extends to a distance distally of the handle 30 and to a position at or near the medical system delivery device distal end 13. It should be understood that, by describing the middle section 40' as passing through the handle 30, the middle section 40' does not necessarily need to pass proximally through the entire length of the handle 30, such as in an embodiment (by way of example and not by way of limitation) where the proximal end 40' of the inner compression member 41 is secured to a distal portion of the cannula 23 and/or the stylet distal end 20" extending within the handle chamber 31 to a position at or near the handle restraint 33.

In addition to holding a threaded tapered plug 21 and optionally the proximal end 40 of the inner compression member 41, the threaded tapered receptacle 22 may secure the proximal portion of the optional cannula 23. Glue 28' may be used at or near an interface of the cannula 23 and distal aperture of the threaded tapered receptacle 22. The glue 28' serves many functions, such as to keep dust from settling within the threaded tapered receptacle 22, to make the cannula 23 easier to clean, and to give aesthetics and a smooth feel to the device.

The handle 30 slidably receives the distal portion of the cannula 23 within the handle aperture 30' and handle chamber 31. As a result, the handle 30 is slidable relative to the stylet 20 (e.g., slidable relative to the threaded tapered plug 21, threaded tapered receptacle 22, and the cannula 23). In use, the physician grips the handle 30 in one hand and grips the stylet 20 (e.g., the receptacle 22) in the other hand. The physician holds the stylet 20 relatively stationary, which prevents the inner compression member and inner guide channel member and its stent carrying portion from moving proximally, and then withdraws the handle 30 proximally relative to the stationary stylet 20 and inner compression member 41. As a result, the physician is thereby retracting an outer guide channel member (discussed below) of the distal end 13 of the delivery system 10 to expose and, ultimately, to deploy a stent locatable at the distal end 13 of the delivery system 10. The handle 30 is in communication with-directly or indirectly through intervening parts-the outer guide channel member at the distal end 13.

As shown in Figure 2, some of those optional parts may include the following: a first bushing 36 having an optional first bushing flange 35; a second bushing 36' having an optional second bushing flange 35'; an intermediate seal 37 intermediate the first and second bushing flanges 35, 35', respectively; a second seal 37' intermediate the second bushing flange 35' and a check flow body 38; and a detachable cap 39, such a Luer cap by way of example but not by way of limitation. In one embodiment, one or both of the intermediate seal 37 and the second seal 37' is from a class such as an O-ring. In another embodiment, one or both of the intermediate seal 37 and the second seal 37' is a cylinder or disk with a center aperture, and may be made from material that comprises an O-ring. The bushings 36, 36' are hollow plastic or metal tubes that take up space within the handle 30 so that the inner compression member has less room to buckle. Fully assembled in one embodiment, the first bushing 36 is inserted within the cannula 23 and the first bushing flange 35 is distal to and abutting the handle restraint 33, which is sized to interfere with the bushing flange 35 to prevent the bushing flange 35 from moving proximal to the handle restraint 33. The second bushing flange 35' is distal to and optionally abutting the bushing flange 35 so to prevent it from moving proximal the first bushing flange 35, and the second bushing 36' is inserted within an opening 139 of the check flow body 38. The intermediate seal 37 and the second seal 37' help to prevent fluids that could be used with the device (discussed below) from entering the handle chamber 31, which directs fluids distally, which fluids may be conveyed through an outer sheath 50 of the middle section delivery device 14 and distal end 13. In one embodiment, the handle restraint 33 is from a class such as a counterbore wherein the restraint 33 comprises, by way of example only and not by way of limitation, a flat-bottomed cylindrical enlargement of the handle chamber 31 sized for receiving a first bushing flange 35, an intermediate seal 37, a second bushing flange 35', and/or a check flow body proximal mating end 38" intermediate the restraint 33 and the handle distal aperture 30".

The handle 30 and check flow body 38 operatively couple with the handle distal aperture 30" receiving a check flow body proximal mating end 38" and being secured together by any suitable means, including but not limited to a crimp, friction fit, press fit, wedge, threading engagement, glue, adhesives, resins, welding (laser, spot, etc.), soldering, brazing, adhesives, chemical bonding materials, or combinations thereof. In one embodiment, the handle 30 comprises a coupling member 32 and the check flow body proximal mating end 38" comprises a coupling member 32', the coupling members 32, 32' being complementary to hold the handle 30 and check flow body proximal mating end 38" together. In one embodiment, the coupling members 32, 32' may form complementary threads. If it is desired to achieve quicker assembly for manufacturing purposes, then the coupling members 32, 32' may be an array of circumferential ridges that form an interference fit when pressed together. If a one-time snap fit is desired, then the coupling members 32, 32' may be circumferential ridges in the form of barbs. In another embodiment, the handle 30 and check flow body proximal mating end 38" may be put together and taken apart for servicing, in which case the coupling members 32, 32' may be circumferential ridges in the form of knuckle threads (e.g., circumferential ridges forming complementary undulating waves). The operatively coupled handle 30 and check flow body proximal mating end 38" according to these embodiments may be fixed such that they do not rotate relative to each other, or may rotate while preventing undesired axial separation.

During use, the detachable cap 39 may be detached or opened and the device flushed with saline to remove air in order to help keep air out of the patient. The intermediate seal 37 and the second seal 37' ensure that any flushed fluid moves distally in the device and does not back up into the handle 30, such as between the handle restraint 33 and the first bushing 36, into the handle chamber 31, or out the handle proximal aperture 30'. The detachable cap 39 (such as a Luer cap) keeps saline from backing out of the check flow body 38, air from flowing into the check flow body 38, and blood from rushing out during periods of high blood pressure inside the patient.

The medical device delivery systems 10 may be used to deploy an implantable prosthesis that is a balloon expandable or self-expanding stent, prosthetic valve device, or other implantable articles provided on the distal end of a delivery system. In operation, a physician inserts the distal end and at least a portion of the middle section delivery device into a vessel passageway, and advances them through the vessel passageway to the desired location adjacent the target site within the vessel passageway of a patient. In a subsequent step, the physician moves the handle proximally, which withdraws the outer sheath and/or the outer guide channel member and releasably exposes the stent for deployment. In another step, the physician inflates the expandable member, such as a balloon, positioned under the stent inner surface to plastically deform the stent into a substantially permanent expanded condition. The physician may inflate the expandable member by injecting fluid such as saline from a syringe into the inner compression member 41, via pusher stylet 20, through a Luer fitting at the proximal end 20'. Therefore, the fluid is directed distally to the expandable member, filling the expandable member chamber and expanding the stent. The physician then deflates the balloon and removes the catheter or delivery device from the patient's body.

In one embodiment as shown in Figure 2, the handle 30 further comprises a check flow body distal mating end 38' and a connector cap 39' (optionally detachable) secured to the check flow body distal mating end 38', and a strain relief 29. In one embodiment, the connector cap 39' is from a class of fasteners such as nuts, and in one embodiment is a flare nut. The connector cap 39' functions to hold (or assist in holding in combination with the check flow body distal mating end 38') a flared proximal portion of an outer sheath 50 and/or a flared strain relief 29 disposed about (and optionally extending proximally from) that held portion of the outer sheath 50. The strain relief member 29 provides a kink resistant point where the outer sheath 50 connects to the connector cap 39' and/or the check flow body distal mating end 38'.

The check flow body distal mating end 38' and connector cap 39' may be operatively coupled mechanically, chemically, and/or chemical-mechanically. In one embodiment, the connector cap 39' is crimped, friction fitted, press fitted, and/or wedged into engagement onto the check flow body distal mating end 38'. In another embodiment for example, the check flow body distal mating end 38' and connector cap 39' are operatively coupled by glue, adhesives, resins, welding (laser, spot, etc.), soldering, brazing, adhesives, chemical bonding materials, or combinations thereof.

According to Figure 2A, yet another embodiment of the connector cap 39' comprises a handle first connector 130 and the check flow body distal mating end 38' comprises a handle second connector 132. According to Figure 2A, the handle first and second connectors 130, 132, respectively, function to operatively couple a strain relief member 29 operatively coupled to the proximal end of the outer sheath 50 (discussed below). In one embodiment, the handle first connector 130 is from a class of fasteners such as nuts, and in one embodiment is a flare nut. Optionally, the distal portion of the second bushing 36' is sized (but for the second bushing flange 35') to be received within a check flow body proximal opening 139 in communication with the second connector 132.

Figure 2A shows an exploded longitudinally sectioned side view of one embodiment of a portion of the handle comprising a first connector 130 and a second connector 132. The handle first connector 130 further comprises a proximal end 134 and a distal end 136. An opening 138 at the proximal end 134 and an opening 140 at the distal end 136 and define a lumen 133 therebetween. There is an engaging surface 142 at or near the distal end 136. A threaded first piece 146 is disposed within the lumen 133 and intermediate the handle first connector distal end opening 140 and proximal end opening 138. The handle second connector 132 further comprises a proximal end 135 and a distal end 137. An opening 141 at the distal end 137 and check flow body proximal opening 139 (e.g., Fig. 2) at the proximal end 135 define a lumen 131 therebetween. There is an engaging surface 143 at or near the distal end 137. A threaded second piece 145 is disposed on the outside surface and intermediate the handle second connector distal end opening 141 and the check flow body proximal opening 139.

According to one embodiment shown in Figures 2A and 2B, the second connector distal end 137 is received within the first connector proximal end opening 138. The first connector 130 and second connector 132 are operatively coupled by a threading engagement between the first connector threaded first piece 146 and the second connector threaded second piece 145. Alternatively, the first connector 130 and second connector 132 are operatively coupled mechanically, chemically, and/or chemical-mechanically. In one embodiment for example, the first connector 130 and second connector 132 are crimped, friction fit, press fit, and/or wedged into engagement. In another embodiment for example, the first connector 130 and second connector 132 are operatively coupled by glue, adhesives, resins, welding (laser, spot, etc.), soldering, brazing, adhesives, chemical bonding materials, or combinations thereof.

Figure 2B shows the second connector threaded second piece 145 operatively coupled to the first connector threaded first piece 146 such that the second connector proximal end 135 is proximal to the first connector proximal end 134 and the second connector distal end 137 is located at or near the first connector distal end 136. As shown in Figure 2B, the second connector engaging surface 143 is spaced proximal to the first connector engaging surface 142 for receiving and compressing a strain relief member second end portion therebetween.

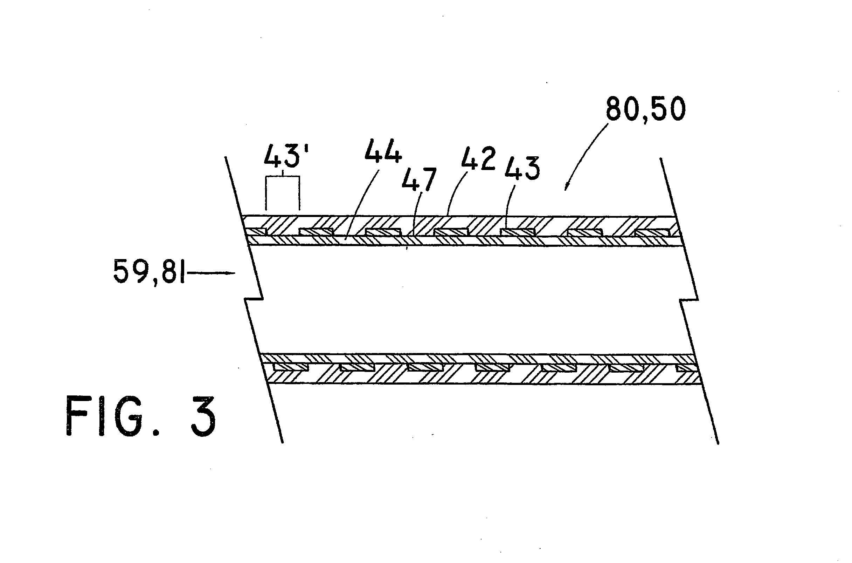

Figure 2C shows one embodiment of an optional strain relief member 29 comprising a tubular first end portion 118 and a flared second end portion 117. According to Figure 2C, the medical device delivery system includes an elongate outer sheath 50 (Figs. 3, 4, 5, 6, 7). Like elements from the previous drawings, embodiments, and description from above are labeled the same. The term elongate is used, not lexicographically but instead, to describe embodiments according to the embodiment that measures at least about 50.0 cm or measures within one of the ranges of lengths exceeding 50.0 cm and as more fully discussed above.

More particularly, Figure 2C shows that the outer sheath 50 comprises a proximal end 57 and a distal end 58. The distal end 58 comprises an opening 52 and the proximal end 57 comprises an opening 53; the openings define a passageway 59 therebetween. In one exemplary embodiment according to Figure 2C, the strain relief member tubular first and second end portions 118, 117, respectively, are disposed about and operatively coupled to the outer sheath proximal end 157. In another embodiment, the tubular first end portion 118 disposes about the outer sheath proximal end 157 while the flared second end portion 117 extends proximally from outer sheath proximal end 157. In addition, the strain relief member second end portion 117 and/or outer sheath proximal end 57 comprise an opening 123 in fluid communication with the outer sheath passageway 59.

By way of example only and not by way of limitation, the terms "operatively coupling," "operatively coupled," "coupling," "coupled," and variants thereof are not used lexicographically but instead are used to describe embodiments of the invention having a point, position, region, section, area, volume, or configuration at which two or more things are mechanically, chemically, and/or chemical-mechanically bonded, joined, adjoined, connected, associated, united, mated, interlocked, conjoined, fastened, held together, clamped, crimped, friction fit, pinched, press fit tight, nested, wedged, and/or otherwise associated by a joint, a junction, a juncture, a seam, a union, a socket, a melt bond, glue, adhesives, resins, welding (laser, spot, etc.), soldering, brazing, adhesives, chemical bonding materials, implanted arrangement, or combinations thereof.

Figure 2C shows the strain relief member second end portion 117 and/or outer sheath proximal end 57 being operatively coupled between the first connector 130 and the second connector 132, and the second connector lumen 131 being in fluid communication with the outer sheath passageway 59. In one embodiment, the strain relief member second end portion 117 and/or outer sheath proximal end 57 comprises a first opposing surface 124 and a second opposing surface 125. The first connector engaging surface 142 is disposed against the first opposing surface 124 and the second connector engaging surface 143 is disposed against the second opposing surface 125, whereby the strain relief member second end portion 117 and/or outer sheath proximal end 57 becomes operatively coupled between the first and second connector engaging surfaces 142, 143, respectively. In one embodiment, the operatively coupled strain relief member second end portion 117 and/or outer sheath proximal end 57 is compressed (e.g., sandwiched) between the first and second connector engaging surfaces 142, 143.

Thus, the check flow body 38 provides an optional three way connector. The check flow body proximal mating end 38" and handle coupling member 32 are operatively coupled. The side port is controlled by the detachable connector cap 39. The body distal mating end 38' is operatively coupled to a second connector cap 39', or optionally the handle second connector 132 is received within and operatively coupled to a handle second connector cap 130.

The foregoing description of a proximal end 12 of a medical device delivery system 10 according to one embodiment of the invention may be one assembly during shipping, or may include a two-part assembly or more. Otherwise stated, the stylet 20 and handle 30 may be sold already combined or may be combined after purchase by inserting the stylet cannula 23 into the handle at the hospital via the threaded tapered plug 21 and threaded tapered receptacle 22. An optional safety lock 34 helps to ensure against unintentional actuation by preventing distal movement of the stylet distal end 20" by extending inwardly within the handle chamber 30 through a slot in the handle outer wall distal to the handle proximal aperture 30'. Consequently, the optional safety lock 34 thereby maintains the handle 30 in an undeployed position until the physician is ready to deploy an implantable prosthesis (e.g., a self-expanding, balloon expandable, or non-expanding stent; prosthetic valve devices, and other implantable articles) at a selected location inside a patient's body.

### MIDDLE SECTION DELIVERY DEVICE 14

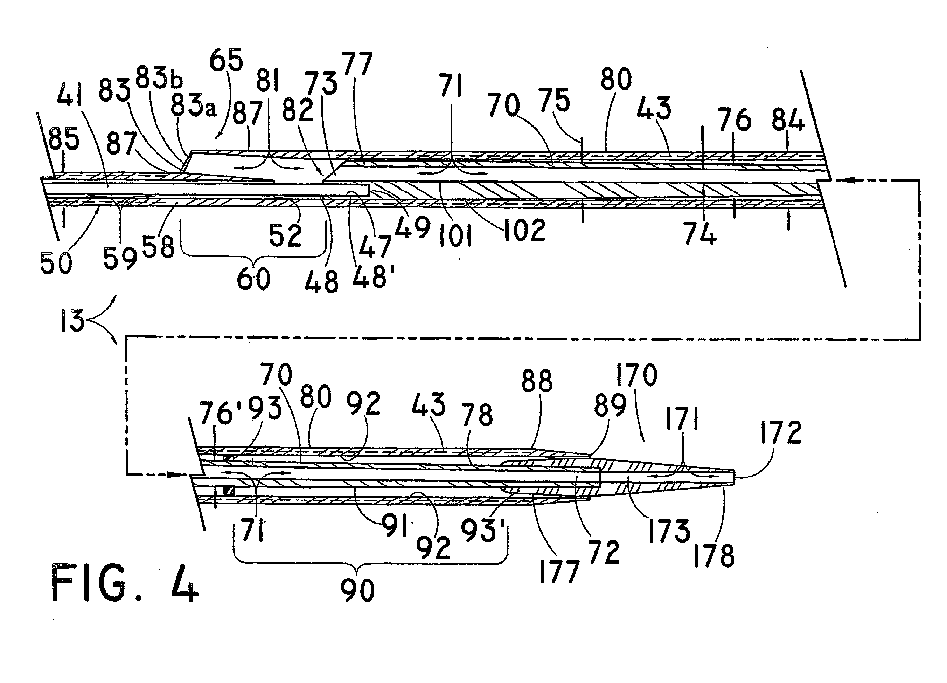

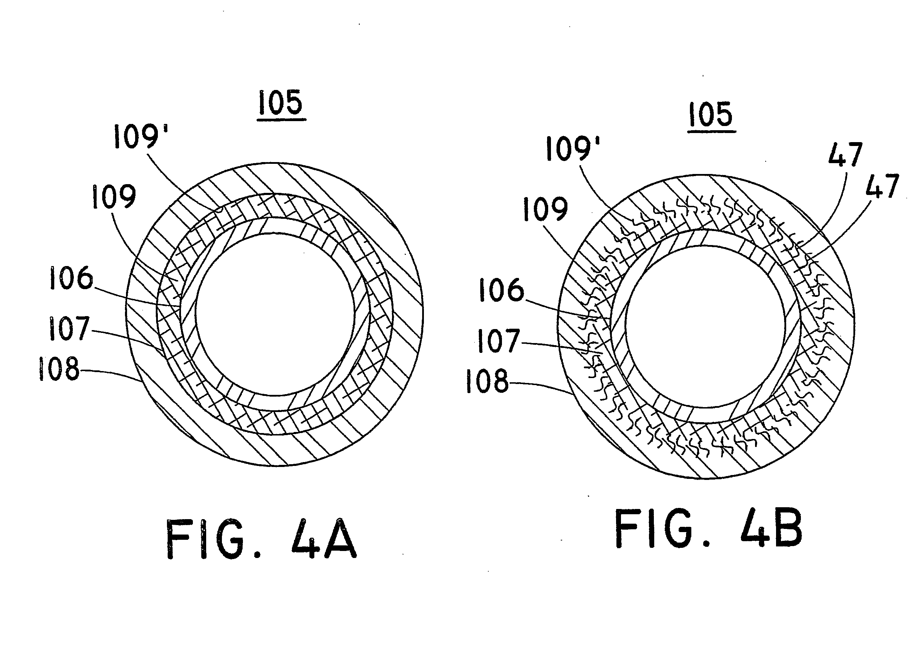

A delivery system 10 as shown in Figures 1 and 2 comprises a middle section delivery device 14. The middle section delivery device 14 is intermediate the proximal end 12 (Figs. 1, 2) and the distal end 13 (Figs. 1, 2) of the delivery system 10. The term "intermediate" is intended to describe embodiments of the invention whereby the middle section delivery device 14 is intermediary, intervening, lying or occurring between two extremes, or spatially in a middle position, state, or nature-though not necessarily equidistant-between the distal tip of the distal end 13 and the proximal tip of the proximal end 12. Furthermore, the middle section delivery device 14 may overlap or be partially inserted into a portion of the distal end 13 and/or the proximal end 12. In another embodiment, a portion of the middle section delivery device 14 (such as the sheath 50 explained below) and the distal end outer guide channel member 80 (discussed below; see Figs. 4, 5, 6, 7) may be an elongate tubular catheter or Flexor^{®} sheath of integral construction.

According to the invention, a middle section delivery device 14 is a flexible, elongate (long, at least about 50.0 centimeters ("cm")) tubular assembly. In one embodiment, the middle section delivery device 14 is from approximately 100.0 centimeters ("cm") to approximately 125.0 cm for use when placing a distal end 13 of the invention within a patient's body, although it may be sized longer or shorter as needed depending on the depth of the target site within the patient's body for delivering the stent. The term "tubular" in describing this embodiment includes any tube-like, cylindrical, elongated, shaft-like, rounded, oblong, or other elongated longitudinal shaft extending between the proximal end 12 and the distal end 13 and defining a longitudinal axis. As used herein and throughout to describe embodiments of the invention, the term "longitudinal axis" should be considered to be an approximate lengthwise axis, which may be straight or may at times even be curved because the middle section delivery device 14, for instance, is flexible and the distal end 13 also may be substantially or partially flexible.

A middle section delivery device 14 comprises an outer sheath 50 (e.g., Figs. 2, 2C, 5, 6, 7). Figure 2C shows that the outer sheath 50 is generally tubular and comprises a proximal end 57 and a distal end 58 and defining a passageway 59 therebetween (e.g., Fig. 2C). In one embodiment, the distal end 58 comprises an opening 52 and the proximal end 57 comprises an opening 53, which openings define the passageway 59. The middle section delivery device 14 further comprises an elongate inner compression member 41 (e.g., Figs. 2, 2C, 5, 6, 7). The outer sheath passageway 59 is configured for slideably receiving the inner compression member 41, a catheter, or other medical device.

Figure 3 depicts an enlarged, longitudinally sectioned view along a partial length of one embodiment of an outer sheath 50 for use as the middle section delivery device 14, with the delivery system's proximal and distal 12, 13 ends, respectively, of the device being removed for clarity. In one embodiment, the outer sheath 50 comprises three layers: an inner layer 44 comprising Teflon material; a middle layer comprising a stainless steel circumferential spiral coil 43; and an outer layer 42 comprising a nylon, a polyether block amide ("PEBA"), and/or other melt bonding material discussed below. The outer layer 42 and inner layer 44 optionally may comprise a lubricious material, one example of which includes a fluorocarbon such as polytetrafluoroethylene (PTFE), to present a slideable surface to allow easier inserting and retracting the middle section delivery device 14 for deploying a self-expanding stent, as will be explained later.

The wall of the inner layer 44 of the outer sheath 50 has sufficient radial rigidity to decrease any tendency of bulging, kinking, and the like under an internal radial expansile force. In other words, the inner layer 44 resists an inner object from protruding or becoming embedded into the inner layer 44, which is beneficial to the slideability of an outer sheath 50. The coil 43 may be compression fitted or wound around the inner layer 44. The coil 43 includes a plurality of turns, and preferably includes uniform spacings 43' between the turns of the coil 43. The coil 43 may be formed of any suitable material that will provide appropriate structural reinforcement, such as stainless steel flat wire or biologically compatible metals, polymers, plastics, alloys (including super-elastic alloys), or composite materials that are either biocompatible or capable of being made biocompatible.

Although the embodiment in Figure 3 shows a flat ribbon shaped wire coil 43, coils of other cross-sectional dimensions, such as round wire, may also be used. When flat wire stainless steel is used, the coil 43 is optionally formed from wire that is about 0.0076 cm (0.003 inches) thick by about 0.03 cm (0.012 inches) wide. In one embodiment, the turns of coil 43 are uniformly spaced 43' apart by approximately 0.03 cm (0.0118 inches). While Figure 3 shows an embodiment that uses coils 43 having uniformly spaced turns and a constant pitch, this is not required and coils 43 may be spaced 43' by non-uniform distances or at varying distances. In one embodiment, the ends of coil 43 are positioned approximately 0.197 inches proximal to the distal end 13 and approximately 0.591 inches distal to the proximal end 12.

The outer sheath 50 for use with the middle section delivery device 14, and the outer guide channel member 80 (Figs. 4, 5, 6, 7) and/or the inner guide channel member 70 (Figs. 4, 5, 6, 7) for use with the distal end 13, are available for purchase from Cook Incorporated, of Bloomington, Indiana under the trade name of "Flexor^{®}." Examples of the Flexor^{®} sheath devices, materials, and methods of manufacturing them are found in United States Patent Nos. 5,700,253 and 5,380,304. The Flexor^{®} sheath is particularly suited for the outer sheath 50 of the middle section delivery device 14 and/or the outer guide channel member 80 of the distal second end 13 due to its thin PTFE liner on the inside wall of the inner layer 44, thin flat wire coil 43, and Nylon and/or PEBA overcoat 42 that captures the coil 43 and PTFE liner 44 and binds the structure together. The PTFE inner layer 44 of the Flexor^{®} sheath resists an expansile inner object from protruding or becoming embedded into the inner layer 44 and, thereby, provides a slick, smooth surface that slides (e.g., across the surface of a stent if the Flexor^{®} sheath is used with the distal end 13 or across the surface of an inner compression member 41 if the Flexor^{®} sheath is used with the middle section 14) relatively easily when retracted to expose, release, and deploy the stent or allow the outer sheath 50 to move relative to the inner compression member 41, and the outer guide channel member 80 to move relative to the inner guide channel member 70, during deployment of the stent.

As an alternative to purchasing the outer sheath 50 for use with the middle section 14 and the outer guide channel member 80 for use with the distal end 13 from Cook Incorporated, one may manufacture the outer sheath and outer guide channel member from various component parts. For instance, one may purchase a tubular inner layer 44 comprising a lubricious material comprising a fluorocarbon such as polytetrafluoroethylene (PTFE or Teflon) from Zeus, Inc. in Orangeburg, South Carolina, and dispose that inner layer 44 over a mandrel. Alternatively, a sheet of material comprising Teflon may be positioned on a mandrel and formed into a tubular body for the inner layer 44 by any suitable means known to one skilled in the art.

The tubular inner layer 44 (whether formed from a sheet on a mandrel or purchased as a tube and slid onto a mandrel) may be slightly longer than the desired length described above for the outer sheath 50 and/or outer guide channel member 80, and slightly longer than the mandrel. In one embodiment, the tubular inner layer 44 may extend about 5.0 cm from each mandrel end. As explained below, the "loose" ends of the tubular inner layer 44 help during manufacturing of the device.

The mandrel-tubular inner layer 44 assembly is prepared for a middle layer comprising a stainless steel circumferential spiral coil 43 as described above and available for purchase from Cook Incorporated or Sabin Corporation in Bloomington, Indiana. As purchased, the coil 43 comes in a long, pre-coiled configuration and will be cut by hand or machine to the desired length either before or after winding the coil about the inner layer 44 to the desired length. As an alternative, one may manufacture the coil from raw material available from Fort Wayne Medical in Fort Wayne, Indiana, and process it into a spiral coil 43 shape.

The operator may apply the spiral coil 43 about the mandrel-tubular inner layer 44 assembly by hand or machine. If by hand, then an end of the spiral coil 43 may be started onto the tubular inner layer 44 by any suitable means, for example, such as hooking and winding (e.g., wrapping) the coil 43 around the tubular inner layer 44 in a pigtailed manner at an initial position a desired distance (e.g., 5.0 cm or more) from a first end of the tubular inner layer 44 and to a terminating position that is a desired distance (e.g., 5.0 cm or more) from a second end of the tubular inner layer 44, and then cutting the coil 43 at the terminating position before or after hooking the coil 43 onto the inner layer 44. If by machine, then chucks, for instance, may hold the opposing ends of the mandrel-tubular inner layer 44 assembly while the spiral coil 43 is threaded through an arm on a machine and started onto the tubular inner layer 44 at the initial position as described above. As the chucks rotate, the inner layer 44 rotates, and the arm moves axially down the length of the inner layer 44, thereby applying the coil 43 in a spiral configuration about the inner layer 44. The machine arm moves to a terminating position where the machine or operator cuts the coil before or after hooking the coil 43 onto the inner layer 44.

An operator then applies an outer layer 42 about the coil-inner layer-mandrel assembly. The outer layer 42 may comprise a polyether block amide, nylon, and/or a nylon natural tubing (individually and collectively, "PEBA" and/or "nylon"). The outer layer 42 preferably has a tubular configuration that disposes about (e.g., enveloping, surrounding, wrapping around, covering, overlaying, superposed over, encasing, ensheathing, and the like) a length of the coil-inner layer-mandrel assembly.

Heat shrink tubing, available from many suppliers, including Zeus, Inc. in Orangeburg, South Carolina for instance and also Cobalt Polymers in Cloverdale, California, may be disposed about the outer layer-coil-inner layer-mandrel assembly. Heating the assembly causes the outer layer 42 to melt. The inner surface of the outer layer 42 thereby seeps through spaces 43' in or between middle layer coils 43 and bonds to both the outer surface of the inner layer 44 and the coils 43. In one embodiment, the inner surface of the outer layer 42 forms a melt bond 47 (explained below) to the outer surface of the inner layer 44. Upon cooling, a solid-state bond results such that the assembly comprises the three layers discussed above. The operator removes the shrink wrap (e.g., by cutting) and withdraws the mandrel. The operator may cut the Flexor^{®} sheath to a desired length for an outer sheath 50 and/or outer guide channel member 80.

The temperature, total rise time, and dwell time for the heat shrink-outer layer-coil-inner layer-mandrel assembly will vary depending on many factors including, for instance, the actual melt bonding material that the outer layer 42 comprises, and also the diameter of the desired Flexor^{®} sheath. For example, the baking parameters for a 2.5 French Flexor^{®} sheath may be approximately 193°C (380 degrees Fahrenheit) for about five minutes, while the baking parameters for a 4 French Flexor^{®} sheath may be approximately 193°C (380 degrees Fahrenheit) for about six minutes.

As an alternative to a Flexor^{®} sheath, the outer sheath 50 may comprise a construction of multifilar material. Such multifilar material or tubing may be obtained, for example, from Asahi-Intec USA, Inc. (Newport Beach, California). Materials and methods of manufacturing a suitable multifilar tubing are described in Published United States Patent Application 2004/0116833 (Koto et al.) having an Application Serial No. 10/611,664 and entitled, "Wire-Stranded Hollow Coil Body, A Medical Equipment Made Therefrom and a Method of Making the Same". Use of multifilar tubing in a vascular catheter device, for instance, is described in United States Patent No. 6,589,227 (Sonderskov Klint, et al.; Assigned to Cook Incorporated of Bloomington, Indiana and William Cook Europe of Bjaeverskov, Denmark), which is also incorporated by reference.

In addition to the outer sheath 50, the middle section delivery device 14 further comprises an inner compression member 41. The delivery device 14 (and, thus, the outer sheath 50 and inner compression member 41) may be constructed to have any diameter and length required to fulfill its intended purposes.

The outer sheath 50, for instance, may be available in a variety of lengths, outer diameters, and inner diameters. In one embodiment, the outer sheath 50 may have a substantially uniform outer diameter in the range from approximately 2 French to approximately 7 French, and in one embodiment the diameter is from approximately 4 French to approximately 5 French in diameter. Otherwise stated, the outer sheath 50 may range from about 0.0254 cm (0.010 inches) to about 0.2286 cm (0.090 inches) in diameter, and in one embodiment the diameter is approximately 0.0127 cm (0.050 inches). Likewise, the passageway 59 may be available in a variety of diameters. In one embodiment, the inner diameter ranges from about 0.081 (0.032 inches) to about 0.1016 cm (0.040 inches) and in a preferred embodiment the passageway 59 is approximately 0.081 cm (0.032 inches). The diameter may be more or less than these examples, however, depending on the intended vessel passageway for the device. For instance, a larger vessel passageway (e.g., greater expandable inner diameter) may tolerate a bigger device with an outer sheath 50 having a correspondingly greater diameter. Conversely, a narrower vessel passageway may require a thinner outer sheath 50. Likewise, the overall length may vary. In one embodiment, the outer sheath 50 will have a length from about 50.0 cm (or about 19.685 inches) to about 125.0 cm (or about 49.213 inches), and more particularly between about 70.0 cm (or about 27.559 inches) and about 105.0 cm (or about 41.339 inches), and in yet another embodiment the length is approximately 100.0 cm (or about 39.370 inches).

The inner compression member 41 comprises an elongated pusher bar, stiffening member, or stiff polymer that helps to "push" the stent by pushing the stent carrying inner guide channel member at or near the distal end 13 in order to counter the urge for the stent or stent carrying member to move as a result of an outer sheath or outer guide channel member being pulled over the stent; thereby "pushing" holds the stent in place at the desired deployment site within the patient's body. The inner compression member 41 "pushes" the stent by helping to prevent or minimize the inner guide channel member from prolapsing, recoiling, kinking, buckling, or moving; thereby keeping the inner guide channel member's stent platform on which the stent is disposed (discussed later) substantially stationary, for the most part, relative to the proximal retraction of the distal outer guide channel member (discussed below) that exposes and, thus, deploys the stent. The phrase "at or near" as used herein to describe an embodiment of the invention includes a location that is at, within, or a short distance such as about 0.1 cm to about 15.0 cm, although other ranges may apply, for instance from about 0.5 cm to about 10.0 cm.

The overall length of the inner compression member 41 may vary, as desired. In one embodiment the inner compression member 41 has a length from about 50.0 cm to about 175.0 cm, and more particularly between about 75.0 cm and 150.0 cm, and in one embodiment the length is approximately 125.0 cm to about 140.0 cm. A portion of the inner compression member 41 (e.g., the proximal end 40 and/or middle section 40') may be contained within the handle 30 and the stylet 20, as explained above (Fig. 2).

Likewise, the diameter or width of the inner compression member 41 may vary. In one embodiment, the inner compression member 41 has a diameter or width ranging from about 0.0254 cm (0.010 inches) to about 0.0762 cm (0.030 inches) by way of example only and not by way of limitation. In one embodiment, the inner compression member 41 has a diameter or width that is approximately 0.016 inches. The diameter or width may be more or less than these illustrative ranges. For example, a deeper target site within a patient may require a thicker inner compression member 41 for greater pushability, but may tolerate lesser flexibility. In addition, the material that the inner compression member 41 comprises determines whether a smaller and more flexible inner compression member 41 will give suitable flexibility, and also determines whether a wider inner compression member 41 may have the flexibility of a thinner inner compression member 41 made of different material. Furthermore, the inner compression member 41 may have a curved transverse cross-section, such as, for example, a circular cross-section, or it may have a polygonal cross-section, such as, for example, a rectangular cross-section. Alternatively, the transverse cross-section of the inner compression member may include both curved and straight portions. According to one embodiment, the inner compression member 41 may have a nonuniform diameter or width along its length. These various diameters, widths, and cross-sections may occur at the inner compression member proximal end 40, the inner compression member middle section 40', and/or the inner compression member distal mating end 48.

It should be understood that the diameter, width, and/or cross-section of the inner compression member 41 may taper. For example, the inner compression member 41 may taper toward the distal end as taught in the United States Provisional Patent Application filed on January 23, 2006 entitled, "Tapered Inner Compression Member and Tapered Inner Guide Channel Member for Medical Device Delivery Systems" and having an application serial number 60/761,676, and the non-provisional application filed on April 20, 2006 by the same title and claiming the benefit of the filing date application serial number 60/761,676 under 35 U.S.C. § 119(e).

Also, an inner compression member 41 may have an outer surface comprising a lubricious PTFE material and/or an inner surface 44 of the outer sheath 50 may comprise a lubricious PTFE material against the inner compression member 41, in order to allow easy retraction of the outer sheath 50, which is in communication with a distal outer guide channel member to deploy a self-expanding stent, as will be explained later.

Generally, the inner compression member 41 and outer sheath 50 may optionally be approximately the same in length, and the axial length of coil 43 will be less than the length of the inner compression member and outer sheath. In one embodiment, however, the inner compression member 41 comprises a proximal end 40 that extends proximal relative to the outer sheath. In yet another embodiment, the inner compression member extends to a position that is distal the outer sheath. In still another embodiment, the inner compression member 41 stops short of extending all the way to the distal tip of the delivery system 10, and may stop generally from 10 to 40 cm short of the distal tip of the delivery system 10, and in one embodiment it stops approximately 20 to 25 cm short of the distal tip of the delivery system 10, where the distal end of the inner compression member 41 is operatively coupled to a proximal portion of an inner guide channel member.

### SYSTEM DISTAL END 13

Now turning to embodiments of a distal end 13 of medical device delivery systems, Figures 4, 5, 6, and 7 show the distal end 13 to be a relatively tubular body. Given the configuration of vessels, vessel passageways, a working channel of an endoscope, or an external accessory channel device used with an endoscope to be navigated, a mostly tubular distal end with a distal tapered, rounded, chamfered, or arrowhead shape may be better tolerated by the patient. Further, in certain embodiments, the distal portion of the distal end 13 may be soft, rounded, and flexible so as to provide further protection for and care to the patient.

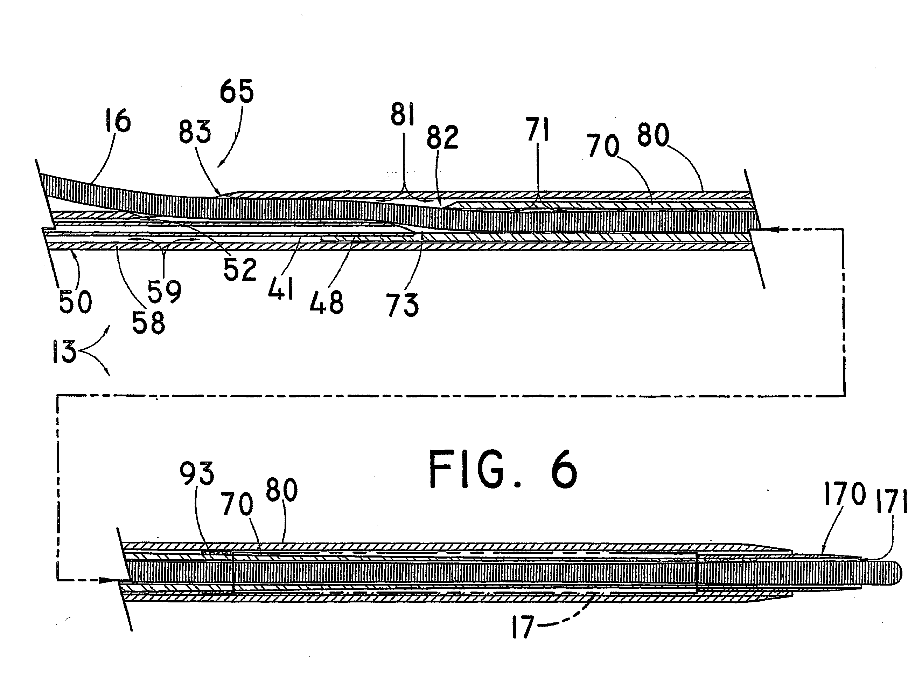

Figure 4 illustrates an embodiment of the distal end 13 of a delivery system for the rapid insertion of self-expanding stents (for example) comprising an inner guide channel member 70, an outer guide channel member 80 axially slideable relative to the inner member 70, a self-expanding deployment device mounting region 90 (e.g., a stent mounting region), and a transition region 60. As used in connection with describing embodiments of the inner and outer guide channel members 70, 80, respectively, the term "guide channel" is understood to be any aperture, bore, cavity, chamber, channel, duct, flow passage, lumen, opening, orifice, or passageway that facilitates the conveyance, evacuation, flow, movement, passage, regulation, or ventilation of fluids, gases, or a diagnostic, monitoring, scope, catheter, other instrument, or more particularly a wire guide (Fig. 6) or another component of the distal end (e.g., an inner member 70 relative to the outer member channel 81).

The distal end 13, according to the delivery system 10 and shown in Figures 4, 5, 6, and 7, may be made of any suitable material (natural, synthetic, plastic, rubber, metal, or combination thereof) that is rigid, strong, and resilient, although it should be understood that the material may also be pliable, elastic, and flexible. By way of illustration only and not by way of limitation, the distal end may comprise one or a combination of the following materials: metals and alloys such as nickel-titanium alloy ("nitinol") or medical grade stainless steel, and/or plastic and polymers such as polyether ether-ketone ("PEEK"), polytetrafluoroethylene (PTFE), nylon and/or a polyether block amide ("PEBA"), polyimide, polyurethane, cellulose acetate, cellulose nitrate, silicone, polyethylene terephthalate ("PET"), polyamide, polyester, polyorthoester, polyanhydride, polyether sulfone, polycarbonate, polypropylene, high molecular weight polyethylene, polytetrafluoroethylene, or mixtures or copolymers thereof, polylactic acid, polyglycolic acid or copolymers thereof, polycaprolactone, polyhydroxyalkanoate, polyhydroxy-butyrate valerate, polyhydroxy-butyrate valerate, or another polymer or suitable material. Where it will not contact the patient (e.g., it is contained within a sheath, working channel of an endoscope, or an external accessory channel device used with an endoscope), the middle section delivery device 14 and distal end 13 do not need to be biocompatible. In contrast, where there is the possibility of patient contact, the material may need to be biocompatible or capable of being made biocompatible, such as by coating, chemical treatment, or the like.

The inner and outer guide channel members 70, 80, respectively, may be made of any suitable material described above for use with the distal end 13. In one embodiment, the inner guide channel member 70 and the outer guide channel member 80 comprise PEEK material, which has the advantage of softening under heat before burning or degrading. PEEK tubing may be purchased from many suppliers, such as Zeus, Inc. in Orangeburg, South Carolina for instance.

Beginning with the inner guide channel member 70, a description will follow relating to features common to embodiments of a distal end 13 of a delivery system 10 for the rapid insertion of "stents" according to the invention. The inner guide channel member 70 is generally tubular and comprises a first end 78 and a second end 77 defining a wire guide channel 71 therebetween. Optionally, the inner guide channel member 70 is configured to be slidably nested, fitted, secured, or otherwise positioned within the outer guide channel member 80 such that at least one of the inner guide channel member first or second ends 78, 77, respectively, is axially intermediate an outer guide channel member first end 88 and an outer guide channel member second end 87.

The first end 78 of the inner guide channel member 70 further comprises a wire guide entry port 72, and the second end 77 has a wire guide exit port 73. The entry and exit ports 72, 73, respectively, define and are in communication via the wire guide channel 71. A port, in describing an embodiment of an inner guide channel member 70 and an outer guide channel member 80 according to the invention, includes any structure that functions as an entry or exit aperture, cutout, gap, hole, opening, orifice, passage, passageway, port, or portal. The inner guide channel member entry port 72 is sized to receive a wire guide into the inner member guide channel 71, and the inner guide channel member 70 is configured so that the wire guide may exit proximally out the inner guide channel member exit port 73. Optionally, the exit port 73 is located at or near the transition region 60. In one embodiment of the present invention, the inner member 70 is a cannula (or catheter) having an entry port 72 and an exit port 73 as previously described and defining a guide channel 71 therebetween.

The inner guide channel member 70 further comprises an outer self-expanding deployment device mounting region 90 (e.g., an outer stent mounting region) positioned intermediate the inner guide channel member entry and exit ports 72, 73, respectively. The length of the inner guide channel member 70 of any of the embodiments of the present invention may vary generally from about 10.0 to about 40.0 cm. In one alternative embodiment, the length of the inner guide channel member 70 is approximately 15.0 to approximately 25.0 cm. In another embodiment, the length of the inner guide channel member 70 is approximately 20.0 cm. Also, the length of the inner guide channel member 70 may depend on the intended stent, and in another embodiment the length of the inner guide channel member 70 is approximately 15.0 cm for an 8.0 cm stent.

The inner guide channel member 70 further comprises inner and outer diameters. In one embodiment, both diameters are substantially uniform over the entire length of the inner guide channel member 70. By way of example, an internal diameter 74 might measure approximately 0.052 cm (0.0205 inches) at or near the inner guide channel member proximal second end 77, at or near the inner guide channel member distal first end 78, and intermediate the first and second ends 78, 77, respectively. Likewise, an inner guide channel member 70 might have an outer diameter 75 that measures approximately 0.109 cm (0.0430 inches). Thus, the outer diameter 75 might measure approximately 0.109 cm (0.0430 inches) at or near the inner guide channel member proximal second end 77, at or near the inner guide channel member distal first end 78, and intermediate the first and second ends 78, 77.

In an alternative embodiment to an inner guide channel member 70 having a substantially uniform outer diameter 75 along its length from about the second end 77 to about the first end 78, the inner guide channel member may also comprise a tapered outer diameter 76. In one embodiment, the inner guide channel member tapers distally to a second outer diameter 76' at or near the inner guide channel member first end 78 or intermediate the inner guide channel member first and second ends 78, 77, respectively. The taper 76 has a decreased cross section, diameter, width, height, area, volume, thickness, and/or other configuration, shape, form, profile, structure, external outline, and/or contour relative to the outer diameter 75. In other words, the inner guide channel member second outer diameter 76' is smaller in cross section, diameter, width, height, area, volume, thickness, and/or other configuration, shape, form, profile, structure, external outline, and/or contour than the outer diameter 75.

Figure 4 further shows an optional atraumatic tip 170 coupled to the inner guide channel member first end 78. Extending distally from the inner guide channel member first end 78, the atraumatic tip 170 is tapered, rounded, chamfered, or arrowhead shape to be better tolerated by the patient. The atraumatic tip 170 comprises a distal first end 178 with a wire guide entry port 172 and a proximal second end 177 with a wire guide exit port 173, whereby the entry and exit ports define an atraumatic tip guide channel 171. The ports 172, 173 and channel 171 are sized to slideably receive a wire guide.

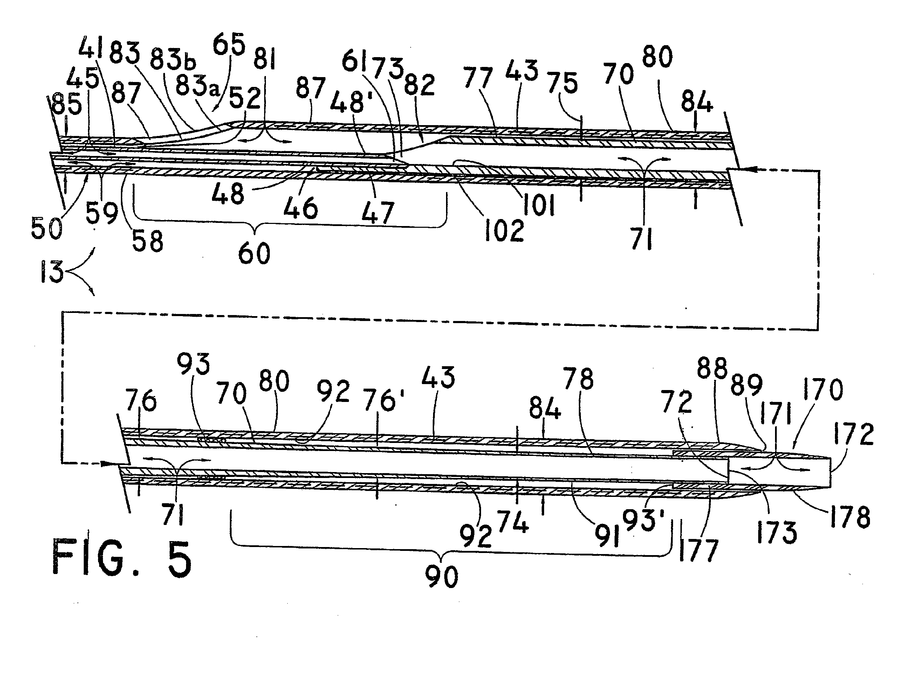

The atraumatic tip second end 177, as shown in Figure 4, may abut the outer guide channel member distal end 88 and, thereby, extend entirely distally beyond a distal opening 89 of the outer guide channel member first end 88. Optionally, the outer guide channel member distal opening 89 is spaced from the atraumatic tip 170 sufficient to allow delivery system to be flushed with saline that exits the distal end to remove air in order to help keep air out of the patient, as explained above. In the alternative and as shown in Figure 5, the atraumatic tip 170 may be configured to have a second end 177 that is beveled such that the atraumatic tip second end 172 is partially positioned within the outer member guide channel 81 and partially proximal to the outer guide channel member distal opening 89. The beveled design of the atraumatic tip second end 177 forms a proximal stop against the outer guide channel member distal opening 89 while permitting the atraumatic tip second end 177 to be partially slidably nested, fitted, secured, or otherwise positioned within the outer guide channel member first end 88 so that the outer guide channel member first end 88 overlaps the atraumatic tip 170 to form a suitable seal that substantially occludes passage of a wire guide between the atraumatic tip 170 and the distal opening 89 of the outer member first end 88 (Fig. 5). Furthermore, the atraumatic tip second end 177 comprises a stent distal restraint 93' as explained below.

In Figure 4, the outer guide channel member 80 also is generally tubular and comprises a first end 88 and a second end 87. The outer guide channel member 80 further comprises a wire guide entry port 82 proximal to the first end 88 and a proximal wire guide exit port 83 located at or near the second end 87. The entry and exit ports, 82, 83, respectively, define a guide channel 81 of the outer guide channel member 80, wherein the ports 82, 83 and channel 81 are sized to slideably receive a wire guide. The entry port 82 is configured to receive a wire guide into the outer member guide channel 81, and in one embodiment, the entry port 82 is defined by the inner guide channel member exit port 73. In that embodiment, the wire guide moves proximally through the inner member guide channel 71 and egresses from the inner guide channel member exit port 73, wherein the proximal passage of the inner guide channel member exit port 73 is designated as the outer guide channel member wire guide entry port 82. The outer guide channel member proximal wire guide exit port 83 is configured so that a wire guide may egress proximally out the outer member exit port 83. In one embodiment, the outer guide channel member distal opening 89 and exit port 83 define the guide channel 81 therebetween.

In one embodiment, the Flexor^{®} sheath, manufactured and sold by Cook Incorporated of Bloomington, Indiana, may be adapted for use with the distal end 13 and/or the middle section delivery device 14. Otherwise stated, the Flexor^{®} sheath, as shown in Figure 3 and described above, may be provided for the distal end 13 and/or the middle section delivery device 14. For instance, the distal end 13 may be constructed as comprising an integral Flexor^{®} sheath tube with the middle section delivery device 14. Alternatively, a Flexor^{®} tubing may be used for either the middle section delivery device 14 or the distal end 13, or both. Then, the separable middle section delivery device 14 and distal end 13 may be attached, adjoined, joined, or combined as taught in the United States Provisional Patent Application filed on April 20, 2005 entitled, "Delivery System and Devices for the Rapid Insertion of Self-Expanding Devices" and having an application serial number 60/673,199, and the non-provisional application filed on April 20, 2006 by the same title and claiming the benefit of the filing date application serial number 60/673,199 under 35 U.S.C. § 119(e), and/or the United States Provisional Patent Application filed on January 23, 2006 entitled, "Melt-Bonded Joint for Joining Sheaths Used in Medical Devices, and Methods of Forming the Melt-Bonded Joint" and having an application serial number 60/761,594, and the non-provisional application filed on April 20, 2006 by the same title and claiming the benefit of the filing date application serial number 60/761,594 under 35 U.S.C. § 119(e).

The Flexor^{®} sheath has a PTFE inner lining 44 that provides a slick, smooth surface for sliding the outer sheath 50 and/or the outer guide channel member 80 proximally. With regard to the distal end 13, the outer guide channel member 80 slides relative to the inner guide channel member 70, and the outer guide channel member inner surface 92 would be the inner layer 44 described above, thereby resulting in minimal friction to a stent 17 on the stent platform 91. The slidable inner surface 92 of the Flexor^{®} sheath exhibits a second benefit of minimizing damage or misalignment to the stent. Indeed, because self-expanding stents continuously exert an expanding force against the inside surface 92 of the outer guide channel member 80, any substantial friction or drag between the stent and the inner surface 92 of the outer guide channel member 80 as the outer guide channel member 80 withdraws may damage the stent or cause the stent to be deployed slightly off of the target site.

The thin flat wire reinforcing coil 43 of the Flexor^{®} sheath provides the outer guide channel member 80 with the necessary radial strength to constrain the stent over long periods of storage time. In contrast, where the inner surface 92 of an outer guide channel member 80 does not comprise the Flexor^{®} sheath inner layer 44 or equivalent, the stent over time may tend to become imbedded in the inner surface 92 and, as a result, interfere with retraction of the outer guide channel member 80 at the time of deployment. In an outer guide channel member 80 that comprises a Flexor^{®} sheath, in addition to the inner layer 44 and the reinforcing coil 43, the outer guide channel member 80 has a Flexor^{®} sheath outer layer 42. The outer layer 42 comprises nylon and/or PEBA to provide the necessary stiffness for pushability, retraction, and control of the outer member 80 to facilitate proper deployment of the constrained self-expanding stent. Therefore, the Flexor^{®} sheath is one non-limiting example of an embodiment of an outer sheath 50 and/or an outer guide channel member 80.

While Figure 4 shows an outer guide channel member 80 having the exit port 83 proximal to the entry port 82 in one embodiment of the outer guide channel member 80, the relative axial distances between the entry and exit ports 82, 83, respectively, vary when the outer guide channel member 80 is in a non-deployed state versus a deployed state, because the outer guide channel member 80 moves axially relative to the inner guide channel member 70. Otherwise stated, Figure 4 shows an embodiment where the exit port 83 is proximal to the entry port 82 in either a non-deployed stent position or in a deployed stent position. In a non-deployed stent position of another embodiment, however, the exit port 83 may be substantially co-planar to or aligned with the entry port 82. In the fully deployed stent position, the exit port 83 may likewise be proximal, co-planar, or aligned with the entry port 82. Optionally, the entry port 82 and exit port 83 are located at or near the transition region 60 to be discussed below.

Furthermore, the outer guide channel member 80 has a stepped 84, 85 profile, whereby the outer guide channel member 80 comprises a first outer diameter 84 intermediate the outer guide channel member first and second ends 88, 87, respectively, and a second smaller outer diameter 85 located at or near the outer guide channel member second end 87 in the vicinity of the transition region 60 and the breech position opening 65. The stepped 84, 85 profile includes an embodiment where the outer guide channel member 80 transitions to the distal end portion 58 of the outer sheath 50 of the middle section delivery device 14. In describing embodiments of the invention, however, the stepped 84, 85 profile shall be discussed in reference to the outer guide channel member 80 in particular, but it should be understood as including a stepped 84, 85 profile in reference to the transition region 60 of the distal end 13 relative to the middle section delivery device 14 where the middle section delivery device 14 and distal end 13 are formed from separate units such as, by way of example only and not by way of limitation, separate "Flexor^{®}" sheaths where one comprises a first outer diameter 84 and the other comprises a second smaller outer diameter 85.

As shown in Figure 4, the second smaller outer diameter 85 of the outer guide channel member 80 is located proximal to the larger first outer diameter 84 and, thereby, comprises a stepped 84, 85 profile. Having a second smaller outer diameter 85 reduces the profile of the outer guide channel member 80 and/or the outer guide channel member 80 transition to the middle section delivery device 14, which is advantageous in procedures involving narrow vessel passageways, endoscope working channels, or accessory channels for use with endoscopes. The difference in the first diameter 84 and the second diameter 85 may vary. By way of illustration, the second diameter 85 may be approximately one-fourth to approximately nine-tenths that of the first diameter 84. In another embodiment, the second diameter 85 may be about one-half that of the first diameter 84. In another embodiment, the first diameter 84 is roughly 5 French while the second diameter 85 is roughly 4 French.

In one embodiment of the stepped 84, 85 profile of the outer guide channel member 80, the second smaller outer diameter 85 is located at or near the outer guide channel member second end 87. The second end 87 may decrease precipitously from the first outer diameter 84 to the second smaller diameter 85. In a precipitous step, the change from the diameters occurs over a short length along the longitudinal axis of the distal end 13. In a further example of a precipitous step, the plane formed by the exit port 83 may be substantially perpendicular to the longitudinal axis of the outer guide channel member 80. In an alternative embodiment, the second end 87 may decrease gradually from the first outer diameter 84 to the second smaller diameter 85. In a gradual step, the change from the two diameters occurs over a length of more than 1.0 millimeter ("mm") along the longitudinal axis of the distal end 13 at or near the transition region 60 and breech position opening 65, and in one instance this change occurs over a length from about 1.0 mm to about 10.0 mm. In a further example of a gradual step, the plane formed by the exit port 83 may be at an angle other than 90 degrees relative to the longitudinal axis of the distal end 13.

Figure 4 also shows a breech position opening 65 located at or near the second end 87 of the outer guide channel member 80 comprising the wire guide exit port 83. In other words, rather than the exit port 83 being an aperture in a lateral sidewall of the outer guide channel member 80 intermediate the first and second ends 88, 87, respectively, in a breech position opening 65 embodiment the exit port 83 is at the rear, back, or proximal part of the distal end 13 at or near the outer member second end 87 and the stepped 84, 85 profile such that it opens in the direction of the outer surface of the outer sheath 50.

The breech position opening 65 may be used for front-loading and the more common procedure of back-loading a wire guide (or catheter, for instance). In a back-loading procedure for a delivery system having a breech position opening 65, the wire guide may pass proximally through the guide channel 71 of the inner guide channel member 70, proximally through the guide channel 81 of the outer guide channel member 80, and leave the exit port 83 of the second end 87 of the outer guide channel member 80 from a breech position opening 65 in a rear, back, or proximal part of the distal end 13. Conversely, in a front-loading procedure for a delivery system having a breech position opening 65, the physician may feed the wire guide distally into a breech position opening 65 at the rear, back, or proximal part of the distal end 13 by entering the exit port 83 of the second end 87 and the guide channel 81 of the outer guide channel member 80 and through the guide channel 71 of the inner guide channel member 70, where the wire guide may exit from the wire guide entry port 72 of the inner guide channel member 70 and/or wire guide entry port 172 of the atraumatic tip 170.

In a distal end 13 having a breech position opening 65 that comprises an exit port 83 located at a breech position of the transition region 60 according to the invention, the wire guide does not need to make any sharp turns away from the longitudinal axis of the distal end 13 that may result in kinking of the wire guide. The breech position opening 65-comprising an exit port 83 according to embodiments of the invention, as those shown in Figures 4, 5, 6, and 7 by way of example and not by way of limitation-is located proximal to the inner guide channel member second end 77 and may be transverse or angled relative to the tubular distal end 13 longitudinal axis. In other words, the wire guide exit port 83 may be positioned at or near a breech position opening 65 of the distal end 13, wherein the exit port 83 is located at or near the rear, back, or proximal part of the outer guide channel member 80 and/or second end 87, rather than being positioned exclusively on the side (e.g., outer circumferential cylinder wall) of the outer guide channel member 80.

In Figure 4, the breech position opening 65 comprises an exit port 83 that is illustrated as being oblique, although other configurations of the exit port may be utilized to aid the wire guide in exiting the rear of the outer member. In one example, the exit port 83 may form a plane substantially perpendicular to the longitudinal axis of the outer guide channel member second end 87. In another example, the plane formed by the exit port 83 may be at an angle other than 90 degrees relative to the longitudinal axis of the distal end 13. Optionally, the oblique exit port 83 of a breech position opening 65 has lateral walls 83a, 83b that act as guide rails to direct a wire guide proximally toward the middle section delivery device 14 and to run along the outside of the outer sheath 50.

The overall axial length of the exit port 83 of the breech position opening 65 may vary. In one embodiment, the length is approximately from about 1.0 mm to about 10.0 mm. Another embodiment has a length of approximately 5.0 mm. The overall width of the exit port 83 may also vary. In one example, the width of the exit port is approximately 1 French. In yet another instance, the width of the exit port 83 ranges from about 1 French to about 4 French. In another example, the width of the exit port 83 may be the approximate difference between the first outer diameter 84 and the second outer diameter 85 of the outer guide channel member 80.

At the transition region 60, the exit port 73 of the inner guide channel member 70 is in communication with the outer guide channel member 80 wire guide entry port 82, while the second end 77 is operatively coupled to the distal mating end 48 of the inner guide channel member 70 as explained below. The length of the transition region 60 may vary. For instance, the transition region 60 may be approximately from about 0.5 cm to about 10.0 cm. In another embodiment, the transition region 60 has the approximate length of about 5.0 cm. Furthermore, the length of the transition region 60 is variable: from a shorter axial length when the outer guide channel member 80 is in a non-deployed axial position; to a greater axial length when the outer guide channel member 80 retracts proximally to deploy the stent. Likewise, the overall length of the transition region 60 varies in the embodiment where the exit port 83 is distal to the entry port 82 when the outer guide channel member 80 is in a non-deployed stent position, compared to the initial length of the transition region 60 in an embodiment where the exit port 83 is proximal to the entry port 82 when the outer guide channel member 80 is in a non-deployed stent position.

In one use of the transition region 60 according to an embodiment of the invention, the outer guide channel member entry port 82 receives a wire guide from the inner guide channel member exit port 73 and the wire guide thereby is received in the outer member guide channel 81. At the transition region 60, the inner member guide channel 71 and outer member guide channel 81 are approximately aligned relatively coaxially in one embodiment. Approximate alignment of the guide channels 71, 81 facilitates a smooth transition of the wire guide. Smooth transition optimally reduces any bending of the wire guide as the wire guide moves proximally from the inner member guide channel 71 to the outer member guide channel 81.

As shown in Figure 4, the distal end 13 also comprises a self-expanding deployment device mounting region 90. This mounting region 90 may be used for implantable prosthesis such as expandable (self-expanding, balloon expandable, or otherwise expanding) and nonexpanding stents, prosthetic valve devices, and other implantable articles for placement inside a patient's body (the implantable prostheses being referred to individually and collectively as "stents" without limiting the invention) and therefore may be referred to as a stent mounting region to include the foregoing implantable prostheses.

The stent mounting region 90 comprises a stent platform 91 on an outside surface of the inner guide channel member 70 located at or near the inner guide channel member second end 78. In describing embodiments of the invention, the platform 91 "at or near" the inner guide channel member second end 78 includes a region intermediate the inner guide channel member entry port 72 and the inner guide channel member exit port 73. The platform 91 may be any stent mounting surface, including but not limited to the outside surface of the inner guide channel member 70, a recess, or an indentation located at or near the first end 78 of the inner guide channel member 70. In a non-deployed state, a self-expanding stent for example (not shown) compresses against the stent platform 91 and disposes around the outside of the inner guide channel member 70.

The stent mounting region 90 controls the lateral movement (e.g., transverse expansion away from the inner guide channel member longitudinal axis) to avoid premature deployment of the stent. In order to control the lateral movement of the stent, the stent is sandwiched between the platform 91 on the inner surface of the stent and the inner surface 92 of the outer guide channel member 80 to keep the stent in a compressed state. Because the stent is bound from above by the inner surface 92 of the outer guide channel member 80 and bound from below by the platform 91 of the inner guide channel member 70, the stent mounting region 90 maintains the stent in a substantially compressed state and controls premature deployment of the stent.

In addition to controlling a stent's lateral movement, the stent mounting region 90 restrains the axial movement of a stent to control the stent movement away from the target site. A proximal restraint 93 controls proximal axial movement of the stent. In one embodiment, the proximal restraint 93 is sized to be large enough to make sufficient contact with the loaded proximal end of the stent without making frictional contact with the inner surface 92 of the outer guide channel member 80. In addition to helping to stop the stent's proximal movement in the non-deployed state, this restraint 93 assists with "pushing" the stent out of the distal end 13 by helping to prevent the inner guide channel member 70 and/or the stent disposed on the stent mounting region 90 from migrating proximally when the outer guide channel member 80 retracts proximally relative to the stationary inner guide channel member 70 in order to expose and deploy the stent. Optionally, the restraint 93 may be radiopaque so as to aid in stent positioning within the vessel passageway at or near the target site within a patient. In one embodiment, an optional distal restraint 93' is large enough to make sufficient contact with the loaded distal end of the stent to control axially distal movement of the stent. Similarly, in another embodiment the proximal second end 177 of an optional atraumatic tip 170 controls the stent's distal axial movement. Indeed, because the medical device delivery system may be used for deploying an implantable prosthesis that comprises balloon expandable or non-expanding stents, prosthetic valve devices, and other implantable articles at a selected location inside a patient's body, the proximal restraint 93 and distal restraint 93' control the axial distal movement of the implantable prosthesis. Optionally, the distal restraint 93' and/or atraumatic tip 170 may comprise radiopaque materials so as to aid in stent positioning within the vessel passageway at or near the target site within a patient.

Figure 4 also illustrates that the inner compression member 41 and inner guide channel member second end 77 may be operatively coupled by any suitable means. In one embodiment, a melt bond 47 (described below) operatively couples an inner compression member distal mating end 48 ("mating end 48") and the second end 77 of the inner guide channel member 70. A melt bond 47 provides surface-to-surface contact between an outer engaging surface 48' of the mating end 48 and the inner guide channel second end 77, thereby forming a more solid connection between the inner compression member 41 and the inner guide channel member 70.

In one embodiment, the inner compression member outer engaging surface 48' may form a melt bond 47 to an inner surface 101 of the inner guide channel member second end 77. Alternatively, the inner compression member outer engaging surface 48' may form a melt bond 47 to the outer surface 102 of the inner guide channel second end 77. In yet another embodiment, the distal mating end 48 of a solid inner compression member 41 as shown in Figure 4 is implanted 49 between (and/or melt bonded 47 between) inner and outer surfaces 101, 102, respectively, of the inner guide channel member second end 77, as taught in United States Provisional Patent Application filed on January 23, 2006 entitled, "Internal Joint for Medical Devices, and Methods of Making the Internal Joint," and having an application serial number 60/761,313, and the non-provisional application filed on April 20, 2006 by the same title and claiming the benefit of the filing date application serial number 60/761,313 under 35 U.S.C. §119(e). In still another embodiment, an insert body operatively couples the inner compression member 41 and the second end inner guide channel member 70, as taught in United States Provisional Patent Application filed on January 23, 2006 entitled, "Internal Cannulated Joint for Medical Delivery Systems," and having an application serial number 60/761,565, and the non-provisional application filed on April 20, 2006 by the same title and claiming the benefit of the filing date application serial number 60/761,565 under 35 U.S.C. §119(e).

As used to describe an embodiment of the invention, melt bonding 47 (for shorthand purposes in describing embodiments according to the invention, melt bonding 47 includes implanting 49) comprises any suitable means for melting, liquefying, softening, making tacky, fusing, or making malleable, pliant, supple, moldable, ductile, or otherwise penetrable by another component or fused to melt bonding material comprising the other element. For instance, melt bonding 47 involves bringing two components together at an interface, wherein one (or preferably both) of the component interfaces are in the melted state. Strictly speaking, true melt bonding 47 requires that both of the components be melted at the interface and that they may be sufficiently chemically and physically compatible such that they fuse together upon cooling.

The melt bonding materials comprising the two components may be the same or substantially same materials. In the alternative, the melt bonding materials may be different, so long as they have substantially similar melting points at standard atmospheric pressure such that the materials soften (or liquefy) under heat and thereby fuse together in a solid state melt bond 47 joining the first and second melt bonding materials of the components. If the materials had melting points that were too different, then one material may degrade or burn and the like before the second material begins to melt.

Melt bonding 47 may be single layer interface whereby one component interface/surface mates to a second component interface/surface, or may be multilayer interface whereby one component is implanted 49 into a second component and then surrounded by the second component. The chemical compatibility can best be expressed in terms of having similar values for surface energy and/or solubility parameter. In simple terms, similar materials tend to have a mutual affinity and a greater propensity to adhere to one another than do dissimilar materials. Melt bonding includes bonding whereby one component is melted while the other component is at or above its melting point.

Melt bonding materials may have different "melt bonding" temperatures at which they soften and become almost tacky without substantial degradation. Melt bonding materials are available from vendors, including Zeus, Inc. in Orangeburg, South Carolina for instance; Cobalt Polymers in Cloverdale, California; and under the trade name of Pebax^{®} PEBA from the Arkema Group. The melt bonding materials may include one or a combination of a class of suitable materials comprising nylon, nylon natural tubing, polyether block amide, polyetheretherketone, thermoplastic, acrylonitrile-butadiene-styrene copolymer, polypropylene, polyamide, ionomer, polycarbonate, polyphenylene oxide, polyphenylene sulphide, acrylic, liquid crystal polymer, polyolefin, polyethylene acrylate acid, polyvinylidene fluoride, polyvinyl, and polyvinyl chloride.

In one embodiment, PEEK material is used for the melt bonding material. PEEK melts at about 334°C (633°F), so the material may be heated from about 331°C (628°F) to about 334°C (638°F) For instance, a radiofrequency loop heater may be used for heating the melt bonding materials. Such a machine is available from Magnaforce, Incorporated and sold under the name and model Heatstation 1500. Another such machine is available from Cath-Tip, Inc. and is sold under the model and name Cath-Tip II. There is a rise dwell and cool down time for the process. The total rise time is approximately 20 seconds and dwell time is approximately 10 seconds. During the dwell time the temperature is approximately 316°C (600°F). In one embodiment where nylon or PEBA are used, heating is at about 204°C (400°F), with dwell time of about 10 seconds.

Figures 4A and 4B present a schematic representation of a cross section 105 of components before and after melt bonding according to one embodiment of the invention. The cross section 105 of Figure 4A, for example, represents an inner component 106, a middle component 107, and an outer component 108. While all components are shown having interfaces in abutting physical contact, they need only be close enough to form a melt bond therebetween. Indeed, as previously explained in connection with the Flexor^{®} sheath's outer layer 42 and inner layer 44, there may even be a middle layer comprising a coil 43 having spacings 43' through which the melt bonding material of the outer layer 42 may move to be into contact with the inner layer 44.

In the example represented in Figure 4A, the middle and outer components 107, 108, respectively, are intended to be melt bonded. The middle component 107 comprises a first melt bonding material 109 while the outer component 108 comprises a second melt bonding material 109', which may be the same material or may be separate materials that have similar melting points at atmospheric pressure.

Figure 4B shows some of the first melt bonding material 109 of the middle component 107 moving into some of the second melt bonding material 109' of the outer component 108. Likewise, some of the second melt bonding material 109' of the outer component 108 moves into some of the first melt bonding material 109 of the middle component 107. It should be noted that both of the first and second materials 109, 109' need not move into the other. Rather, the first and second materials 109, 109' need only bond at an interface, with or without mixing and the like. By way of example, the Flexor^{®} sheath's outer layer 42 may melt to the middle layer coil 43 (which has not melted) and bond to the outer surface of the inner layer 44 with or without the outer surface of the inner layer 44 melting into the outer layer 42.

Figure 4B further shows that the first and second melt bonding materials 109, 109', respectively, of the middle component 107 and the outer component 108 or other components that have been melt bonded, upon cooling to solid state will form a melt bond 47 operatively coupling the components and/or the melt bonding materials that comprise the components. This results in additional strength and helps to form a more solid connection to the melt bonded components, because a solid-state bond results from using a suitable form of heat for melting and solidifying (e.g., fusing and/or cross-linking bonds formed at the melt bonded material interfaces).

Figure 5 illustrates a schematic view showing an alternative embodiment of a distal end 13 of a delivery system for the rapid insertion of stents comprising an inner guide channel member 70, an outer guide channel member 80 axially slideable relative to the inner member 70, a deployment device mounting region 90 (e.g., a stent mounting region), and a transition region 60. Like elements from the previous drawings, embodiments, and description from above are labeled the same. In this embodiment, the inner compression member 41 optionally comprises a passageway 45 (e.g., hollow, having a lumen) that facilitates the conveyance, ventilation, flow, movement, blockage, evacuation, or regulation of medication and/or fluids or accommodates the insertion of a diagnostic, monitoring, scope, or other instrument.

The tubular inner compression member 41 may have a uniform inside diameter ranging from about 0.134 to about 0.335 cm (0.0527 to about 0.132 inches). The wall thickness of the tubular inner compression member 41 is approximately 0.0038 cm (0.0015 inch). These dimensions are illustrative only, and the inner diameter and wall thickness may be constructed to be of any size necessary to accomplish the purposes for which the delivery system is to be employed (i.e., limited by the vessel passageway or working channel in which the device is to be used).

In addition, this inner compression member 41 has an optional distal one-way valve 61. Thus, the valve 61 may serve a dual function. First, a one-way valve is relatively resistant to contamination from bodily fluids entering the inner compression member passageway 45. Second, it allows the movement of medication and/or fluids to exit distally the inner compression member 41 passageway 45 at or near the transition region 60 and may direct medication and/or fluids into the inner member guide channel 71 and/or the outer member guide channel 81.

Indeed, the inner compression member passageway 45 may facilitate using the medical device delivery system for deploying an implantable prosthesis that comprise balloon expandable stents, prosthetic valve devices, and other implantable articles (individually and collectively, "stent") at a selected location inside a patient's body. The stent is disposed at the deployment device mounting region 90 intermediate the proximal restraint 93 and distal restraint 93' to control the axial distal movement of the implantable prosthesis.

In one embodiment for using the delivery system with a balloon expandable implantable prosthesis, the inner compression member distal mating end engaging surface 48' operatively couples to the inner guide channel member outer surface 102 (or is welded to an outer surface of a metal cannula that has the inner guide channel member second end 77 glued within the cannula lumen), and an inflation member (e.g., a balloon) extends distally from the inner compression member distal mating end and is disposed over the proximal restraint 93 and distally about the platform 91 of the stent mounting region 90 such that the balloon is located under the stent. The stent is positioned within the vessel passageway at or near the target site within a patient, wherein the outer sheath 50 and outer guide channel member 80 is axially slideable relative to the inner compression member 41 and inner guide channel member 70 upon corresponding axial slideable movement of the handle 30, thereby exposing and, ultimately, deploying the stent from the stent mounting region 90. The stylet 20 may be adapted to receive a syringe for allowing inflation fluid, such as saline, to travel from and through the proximal end 40 of the inner compression member 41 and out the valve 61 at the distal end 48 in order to fill the inflation chamber of the balloon. Therefore, balloon expands under the stent and, as a result, the stent expands radially to plastically deform the stent into a substantially permanent expanded condition. The physician then deflates the balloon and removes the inner guide channel member 70 and remainder of the delivery system from the patient's body. This description of using the delivery system for balloon expandable implantable prosthesis is given by way of example and not by way of limitation. Alternatively, a tubular inflation fluid carrying device is in the outer sheath passageway 59 and extends from the system proximal end 12 to the system distal end 13 and operatively couples to an inflation member disposed under the stent.

In one embodiment of the distal end 13 of a delivery system illustrated in Figure 5, an internal joint 46 comprises a melt bond 47 that operatively couples the inner guide channel member distal mating end 48 and the second end 77 of the inner guide channel member 70. For example, the inner compression member outer engaging surface 48' may form a melt bond 47 to the inner surface 101 (or alternatively to the outer surface 102) of the inner guide channel member second end 77, as taught above.

The embodiment shown in Figure 5 also illustrates that the exit ports 83 and 73 may have various configurations. First, these exit ports curve, and second, compared to Figure 4 they slope over a longer overall axial length to aid the wire guide in exiting the inner member and outer member, respectively. Furthermore, the exit port 83 thereby has longer axial lateral walls 83a, 83b for acting as guide rails to direct a wire guide proximally toward the middle section 14 and to run along the outside of the outer sheath 50.

Moving to the atraumatic tip 170 as illustrated in Figure 5, this is a little less arrowhead-shaped compared to the atraumatic tip 170 shown in Figure 4. Instead, the atraumatic tip in Figure 5 has a second end 177 that comprises a right cylindrical tubular configuration. Furthermore, the sides of the atraumatic tip second end 177 are more uniformly parallel and do not form a proximal stop against the outer guide channel member distal opening 89 as in a beveled embodiment of the atraumatic tip second end 177 as illustrated in Figure 4. The atraumatic tip second end 177 optionally comprises a stent distal restraint 93' for controlling distal axial movement of the implantable prosthesis when the medical device delivery system is used for deploying balloon expandable or non-expanding stents, prosthetic valve devices, and other implantable articles at a selected location inside a patient's body.

Turning now to Figure 6, that figure shows a partially sectioned distal end 13 in accordance with an embodiment of the device according to Figure 5 with a wire guide 16 inserted therein. In a back-loading procedure, the wire guide 16 enters the guide channel 171 of the atraumatic tip 170 and travels proximally toward the inner guide channel member 70. The wire guide 16 then enters the inner member guide channel 71 and travels proximally toward the outer guide channel member 80 via the entry port 82 and enters the outer member guide channel 81 and out the exit port 83. The less common front-loading procedure could be described as above but conversely stated.

In Figure 6, the inner and outer guide channels 71, 81, respectively, are substantially aligned coaxially along an approximate center longitudinal axis of the distal end 13. Because the channels 71, 81 are substantially aligned, the wire guide 16 moves through the inner member guide channel 71 to the outer member guide channel 81 and out the outer guide channel member exit port 83 at or near the breech position opening 65 with relatively little kinking, bending, buckling, or bowing.

It should be noted that for the ease of showing the wire guide 16, the wire guide 16 proximal to the exit port 83 is shown slightly offset from outer sheath 50, though the wire guide 16 may actually run along the outside of the outer sheath 50 or in a groove (not shown) in the outer sheath 50.

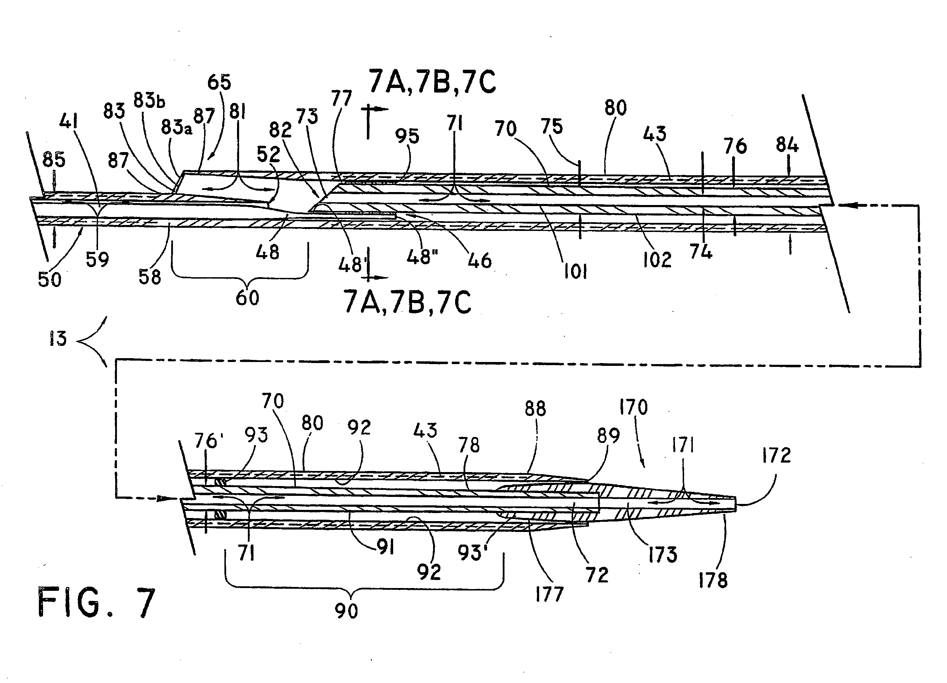

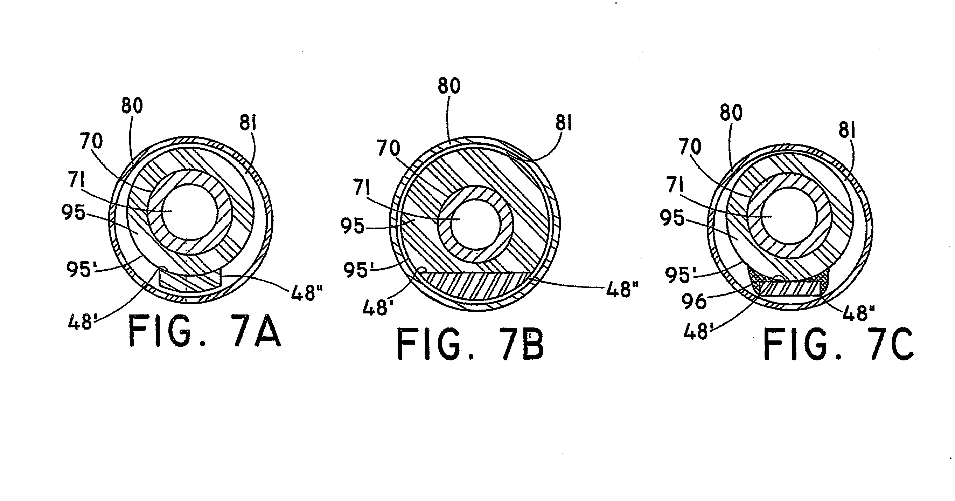

Figure 7 illustrates a schematic view showing an alternative embodiment of a distal end 13 of a delivery system for the rapid insertion of stents comprising an inner guide channel member 70, an outer guide channel member 80 axially slideable relative to the inner member 70, a deployment device mounting region 90 (e.g., a stent mounting region), and a transition region 60. Like elements from the previous drawings, embodiments, and description from above are labeled the same. This embodiment represents an alternative embodiment of a joint 46 for operatively coupling the inner compression member 41 and inner guide channel member 70 with a cannula 95.

In one embodiment, the cannula 95 is a tubular device comprising metal such as medical grade stainless steel or super-elastic alloys (e.g., nitinol) to name but a few non-limiting examples. The cannula 95 is sized for receiving the inner guide channel second end 77 and/or the inner guide channel second end outer diameter 75. The outer surface 102 of the inner guide channel second end 77 is operatively coupled to an inner engaging surface of the securing body 95 by glue, adhesives, resins, chemical bonding materials or combinations thereof and the like (collectively and individually, "glue"). The securing body may be formed as a separate piece.

In addition to securing the outer surface 102 of the inner guide channel member second end 77 to an inner engaging surface of the cannula 95, the cannula 95 also operatively couples the inner guide channel member distal mating end 48. An outer engaging surface 48' of the mating end 48 is in an abutting relationship (e.g., touching, in contact directly or by intervening parts, or adjacent) to an outer engaging surface of the cannula 95. The mating end 48 and cannula 95 welded, soldered, brazed, or fused. In order to minimize some of the sharp edges created by a welded, soldered, brazed, or fused joint, tube may be disposed about the joint 46. In one embodiment, the tube is a melt bonding tube disposed about and melt bonded to the joint 46.

According to Figures 7, 7A, 7B, and 7C, the distal mating end 48 comprises a contoured configuration 48" that is complementary to an outer engaging surface 95' of the cannula 95. Thus, in an embodiment comprising a cannula 95 that is curved or otherwise circular in cross-section, then Figure 7A shows that the contoured configuration 48" is fluted so that the outer engaging surface 48' is capable of being in an abutting relationship (e.g., touching, in contact directly or by intervening parts, or adjacent) relative to a curved or circular outer engaging surface 95' of the cannula 95. A fluted contoured configuration 48" comprises any curved, shoehorn shape, celery shape, semicircular shape, crescent shape, wishbone shape, saddle shape, C-shaped, V-shaped, U-shaped, or other arcuate configuration. In another embodiment, an outer engaging surface 95' of the cannula 95 could have a flat portion, and Figure 7B shows that the contoured configuration 48" is likewise flat so that the outer engaging surface 48' is capable of being in an abutting relationship (e.g., touching, in contact directly or by intervening parts, or adjacent) relative to the flat portion of the outer engaging surface of the cannula 95. Even when the outer engaging surface 95' of the cannula 95 is curved or circular in cross section, however, Figure 7C shows that the inner compression member contoured configuration 48" could be flat, because the soldering, brazing, or fusing may fill in the space between the outer engaging surface 48' and a tangent that the flat configuration 48" forms to the curved portion of the outer surface 95' of the cannula 95. Similarly, if welding 96 is used, then the flat configuration 48" will form to a curved or circular outer engaging surface 95' of the cannula 95.

### JOINT ASSEMBLY FOR OPERATIVELY COUPLING AN INNER COMPRESSION MEMBER AND AN INNER GUIDE CHANNEL MEMBER

The invention as recited the appended claims comprises an internal joint assembly, and method of making the internal joint assembly, for use in a medical device delivery system for deploying an implantable prosthesis (e.g., a self-expanding, balloon expandable, or non-expanding stent; prosthetic valve devices, and other implantable articles) at a selected location inside a patient's body. The invention further comprises a medical device delivery system for deploying an implantable prosthesis.

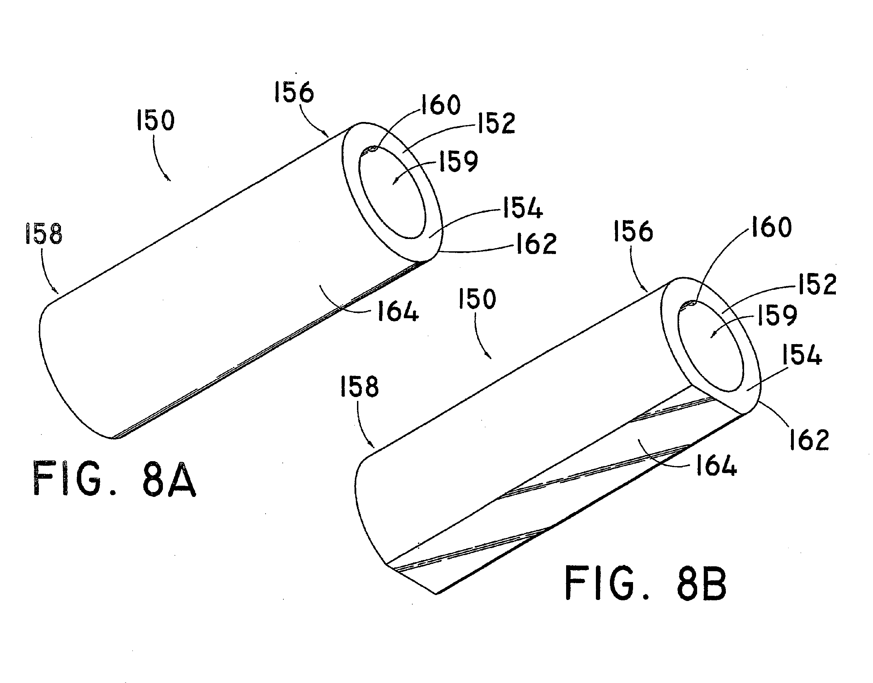

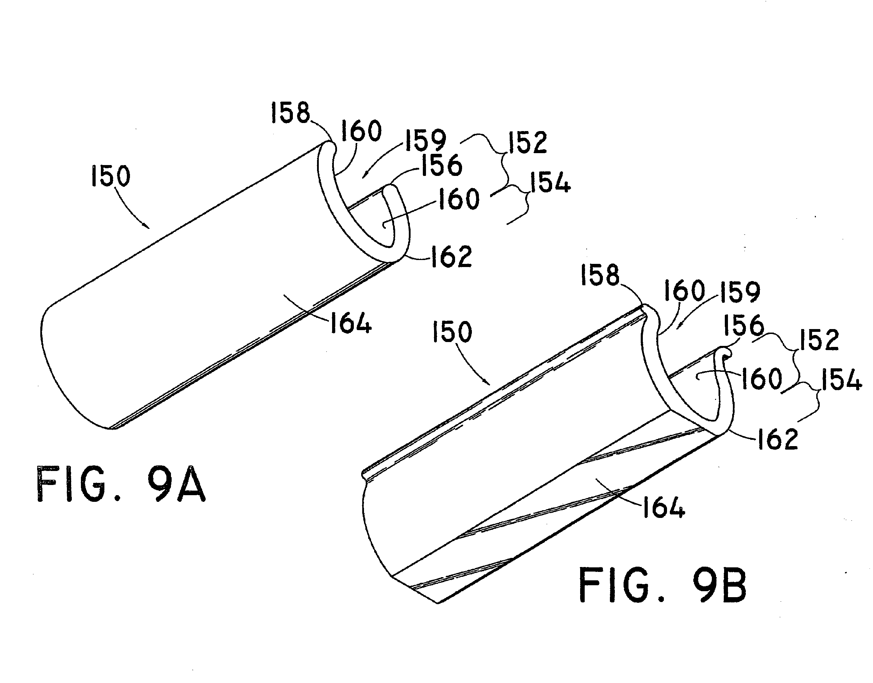

Figures 8A, 8B, 9A, and 9B show alternative embodiments of a securing body 150 according to the invention. In one embodiment, the securing body 150 is a hollow tube, cylinder, cannula (with or without a trocar-like end), or other coupling device having a lumen (individually and collectively, "securing body") (Figs. 8A, 8B) or with a cutout portion to form a clip (Figs. 9A, 9B). In another embodiment, the securing body 150 comprises a cylinder that is circular, elliptical, hyperbolic, parabolic, curved, polygonal, rectangular, or irregular in shape or cross section. In yet another embodiment, the securing body 150 comprises a cannula 95 (Fig. 7) in the form of a hollow right cylindrical body that, within manufacturing tolerance, has a constant radius "r," length "h," uniform inner and outer diameter, and a three-dimensional surface according to the equation in Cartesian coordinates of: (x²/a) + (y²/b) = 1, where a = b.

The securing body 150 may be made of any suitable material (natural, synthetic, plastic, rubber, metal, or combination thereof) that is rigid, strong, and resilient, although it should be understood that the material may also be pliable, elastic, and flexible. One class of suitable materials comprises metals such as stainless steel, nickel titanium alloy, and other alloys (including super-elastic and/or memory shaped alloys). In one embodiment of the securing body 150 according to the invention, the securing body 150 shown in Figures 8A, 8B, 9A, and 9B may comprise metal that is sufficiently rigid to provide sufficient strength to transfer tensile and compressive forces from the inner compression member 41 to an inner guide channel member 70 (Figs. 4-7) while the handle 30 moves the outer sheath 50 and outer guide channel member 80 axially relative to the inner compression member 41 and the inner guide channel member 70. Another class of suitable materials comprises polymers, plastics, and composite materials, such as those identified above for use with the distal end of the delivery system and incorporated herein by reference.

The securing body 150 may be from about 0.5 mm to about 5.0 mm in length, although this length may be greater or lesser as desired. If the securing body 150 is a substantially rigid metal such as medical grade stainless steel, then a greater length would make a stiff region in the distal end 13 of the medical device delivery system, and could impede the distal end 13 from navigating tortuous vessel passageways to a selected location inside a patient's body for deploying the stent. If the securing body 150 comprises a super-elastic alloy, then the securing body 150 may be greater than 5.0 mm in length. Some examples of embodiments of super-elastic alloys include copper-zinc-aluminum, iron-manganese-silicon, gold-cadmium, copper-aluminum, and copper-aluminum-nickel. In one embodiment, the super-elastic alloy is a nickel-titanium alloy ("nitinol," an acronym of Nickel Titanium Naval Ordnance Laboratory, where the alloy's properties were discovered). Nitinol is an alloy containing nearly equal numbers of nickel and titanium atoms, and the relative amounts of nickel and titanium can be varied by a few percent. Nitinol is known for its flexibility.

The inner and outer diameters of the securing body 150 may be from about (0.020 inches) 0.05 cm to about 0.2 cm (0.080 inches), although this length may be greater or lesser as desired. In one embodiment, the inner diameter is approximately 0.096 cm (0.038 inches). In one embodiment, the outer diameter is approximately 0.118 cm (0.044 inches). Although the outer diameter could be greater, it is limited by the inner diameter of the outer guide channel member 80 so as not to cause the securing body 150 to exert pressure or friction against the inner surface 92 of the outer guide channel member 80 and, thereby, possibly impede proximal movement of the outer guide channel member 80. Given the range of permissible inner and outer diameters of the securing body 150, the outer wall of the securing body 150 may have different thicknesses, and in one embodiment is approximately 0.015 cm (0.006 inches) in thickness. A thinner wall tends to provide greater flexibility relative to a thicker wall. Along the length of the securing body 150, the wall has a generally uniform thickness.

The securing body 150 has an inner securing section 152 and a base securing section 154. These securing sections 152, 154 may be spaced apart as needed at various intervals longitudinally along the length of the securing body 150 such that the inner securing section 152 is located longitudinally distally (or proximally) of the base securing section 154. Otherwise stated, when viewed end on, the inner securing section 152 may form the front portion of the securing body 150 while the base securing section 154 forms the rear portion of the securing body 150 or vice versa, by way of example and not by way of limitation.

Alternatively, the sections 152, 154 may be spaced apart radially such that the inner securing section 152 comprises an upper portion of the securing body 150 while the base securing section 154 comprises a lower portion of the securing body 150. The upper and lower portions need not be equal. For example, the inner securing section 152 shown in Figure 8A may comprise from about 25% to about 95% of a cross section of the securing body 150 if there were an imaginary plane that sectioned the inner securing section 152 from the base securing section 154.

From a further spatial perspective, the base securing section 154 of the securing body 150, relative to the inner securing section 152, is nearest the inner compression member distal mating end. Otherwise stated, the base securing section 154 is intermediate the inner securing section 152 and inner compression member distal mating end engaging surface 48'.

In one embodiment, the securing body 150 is elongated, whereby the length is greater than the outer diameter. In another embodiment, the securing body 150 is ring-shaped, whereby length is substantially the same or less than the outer diameter. While in one embodiment the base securing section 154 and the inner securing section 152 are substantially similar in length, in yet another embodiment the base securing section 154 could be greater in length than the inner securing section 152, or vice versa.

As shown in Figure 8A, the inner securing section 152 further comprises a first end portion 156 and a second end portion 158 and defining a receiving cavity 159 therebetween that is sized to receive the inner guide channel second end 77 and/or the inner guide channel second end outer diameter 75 (Figs. 4, 5, 7, 11, 12). The receiving cavity 159 has at least one inner engaging surface 160 disposed about and operatively coupled to the inner guide channel member second end outer surface 102 (Figs. 4, 5, 7, 11, 12).

In other words, the first end portion 156 and a second end portion 158 are configured to serve as a structure that secures a portion of the inner guide channel member within the receiving cavity 159. The inner securing member inner engaging surface 160 is directly abutting (e.g., touching, in contact directly or by intervening parts, or adjacent) the inner guide channel member second end outer surface 102. The receiving cavity inner engaging surface 160 of the inner securing section 154 of the securing body 150 and the inner guide channel member second end outer engaging surface 48' are operatively coupled by any suitable bond as discussed next.

For instance, operatively coupling the inner guide channel member within the receiving cavity 159 between the first and second end portions 156, 158, respectively, of the inner securing section 152 (e.g., Figs. 8A, 8B) may comprise a friction fit, press fit tight, nesting, wedging, or any combination thereof, to name a few. In still another embodiment, operatively coupling the inner guide channel member within the receiving cavity 159 between the first and second end portions 156, 158, respectively, of the inner securing section 152 (e.g., Figs. 9A, 9B) may comprise crimping, clamping, pinchers, teeth, barbs, or any combination thereof, to name a few. The inner securing section inner engaging surface 160 may also be ribbed, have a rubber coating, or other sticky layer in order to increase friction and hold between the inner engaging surface 160 and the inner guide channel member second end outer surface 102.

In an alternative embodiment, the inner securing member inner engaging surface 160 is abutting (e.g., touching, in contact directly or by intervening parts, or adjacent) the inner guide channel member second end outer surface 102 by the use of intervening glue, adhesives, resins, chemical bonding materials or combinations thereof and the like (collectively and individually, "glue"). The engaging surface 160 may also be secured to a periphery of the inner guide channel member second end outer surface 102, or to a coating, sheath, tubing, or cover on the inner guide channel member second end outer surface 102. By way of example of one type of glue, Loctite 4061 instant adhesive may be used to operatively couple the inner securing member inner engaging surface 160 and the inner guide channel member second end outer surface 102. Loctite 4061 instant adhesive is formulated to polymerise rapidly in thin films between two surfaces to form rigid thermoplastics. Loctite 4061 instant adhesive is a medical device adhesive particularly suitable for a wide variety of substrates such as rubber, plastics, and metals, ant it is available from the Loctite Corporation.

The receiving cavity 159 of the first end portion 156 and a second end portion 158 may form a full cylinder such as when the inner securing section 152 comprises a cannula generally in the shape of a hollow tube (e.g., Figs. 8A, 8B). As an alternative, the inner securing section 152 may comprise a partial cylinder with a longitudinal cutout portion of the outer wall of a cannula, whereby the receiving cavity 159 is between the first end portion 156 and second end portion 158 (e.g., Figs. 9A, 9B) that form the shape of a groove, arch, curve, wishbone, saddle, or otherwise has a U-shape, horseshoe shape, Omega-shape, and the like. Thus, the securing body 150 may be generally U-shaped. In this alternative embodiment shown in Figures 9A and 9B, the securing body 150 comprises a clip. As shown in Figures 9A and 9B, the first end portion 156 and second end portion 158 form a clip design having a sufficiently pliable and elastic memory such that that the first end portion 156 and second end portion 158 may spread, bend, flex, and otherwise stretch apart or separate to receive the inner guide channel member second end diameter 75 and then snap onto and hold the inner guide channel member second end outer surface 102 and/or the inner guide channel member second end diameter 75 with the securing body inner engaging surface 160.

In the case of a generally cylindrical configuration of the inner guide channel member second end 77, a nearly rounded or annular cross section of the inner securing section 152 (Figs. 8A, 8B, 9A, 9B) may be better for passing the inner guide channel member second end 77. Thus, the cross-sectional profile of the inner securing section 152 optionally may have circular or annular inner and outer diameters. Other profiles may be utilized, however, because the outer diameter of the inner securing section 152 need not be constant. Examples of other profiles when viewing the inner securing section 152 end-on include a profile that is tapered (reduced circumference), rounded, oblong, rectangular, ovulate, triangular, elliptical, hyperbolic, parabolic, curved, polygonal, rectangular, or irregular in shape or cross section or a combination thereof as specific but non-limiting examples of inner securing section 152 embodiments that include a receiving cavity 159 for receiving and axially holding the inner guide channel member second end 77.

Turning to the base securing section 154 of the securing body 150 shown in Figures 8A, 8B, 9A, and 9B, the base securing section 154 comprises a curved portion 162 and an outer engaging surface 164. The curved portion 162 and/or outer engaging surface 164 may extend longitudinally along the full length of the securing body 150 or, in an alternative embodiment, may comprise a portion that extends less than the full length of the securing body 150.

The outer engaging surface 164 may be the outer or the bottommost boundary, periphery, or exterior of a portion of the securing body 150. The outer engaging surface 164 may be curved (Figs. 8A, 9A). Thus, embodiments of the securing body 150 shown in Figures 8A and 9A may have an outer engaging surface 164 comprising cross sectional shapes that are fluted, whereby fluted comprises any curved, shoehorn shape, celery shape, round, circular, semicircular shape, crescent shape, wishbone shape, saddle shape, C-shaped, V-shaped, U-shaped, or an arcuate configuration that curves radially inward or curves radially outward. In other embodiments shown in Figures 8B and 9B, the outer engaging surface 164 is substantially flat, whereby flat comprises having a horizontal surface without a slope, tilt, or curvature. In still other embodiments in accordance with Figures 8B and 9B, the outer engaging surface 164 is substantially flat in a vertical plane. In yet another class of alternative embodiments in accordance with Figures 8B and 9B, the outer engaging surface 164 may have a tilt, angle, or taper comprising an inwardly or outwardly protruding slope relative to a longitudinal axis of the securing body 150, such as a one-sided beveled structure or working end of a screwdriver in the horizontal plane, vertical plane, or in between those planes, by way of example and not be way of limitation.

In addition, the outer engaging surface 164 of Figures 8A, 8B, 9A, and 9B may be a smooth, even, level surface. In one embodiment, however, mechanical or chemical etching, sandblasting, laser-cutting, machining, milling, drilling, chemically treating, or otherwise preparing the outer engaging surface 164 improves its bonding properties to the outer engaging surface 48' of the inner compression member distal mating end 48 (Figs. 4-7, 10-11, 14-14A). Likewise, the outer engaging surface 48' of the inner compression member distal mating end 48 may be treated in order to improve its bonding properties to the outer engaging surface 164 of the securing body 150. By securing body outer engaging surface 164 and inner compression member distal mating end outer engaging surface 48', it should be understood to include an surface (regular or irregularly shaped, textured, or treated as described above) as well as any coating comprising any substance, compound, molecule, or material (whether comprising a solid, liquid, fluid, gel, gas, or vapor) chemically bonded via covalent bonds, ionic bonds, or intermolecular bonds (such as ion-dipole forces, dipole-dipole forces, London dispersion forces, and/or hydrogen bonding), adhered, or otherwise applied by the method(s) of laminating, taping, dipping, spraying, depositing, vapor deposition, wrapping (thermally fusing together), painting and curing, and the like.

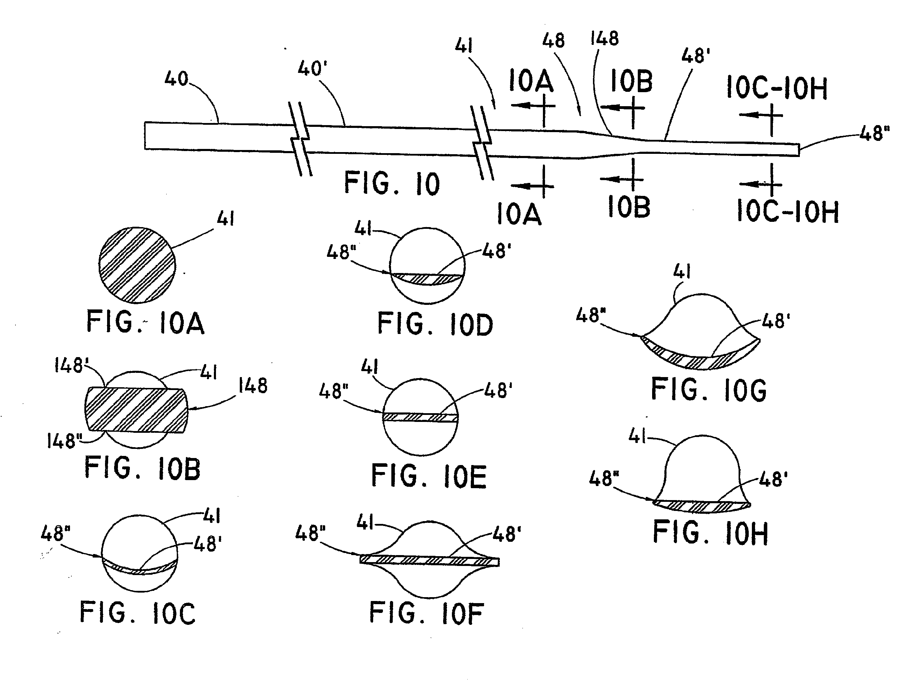

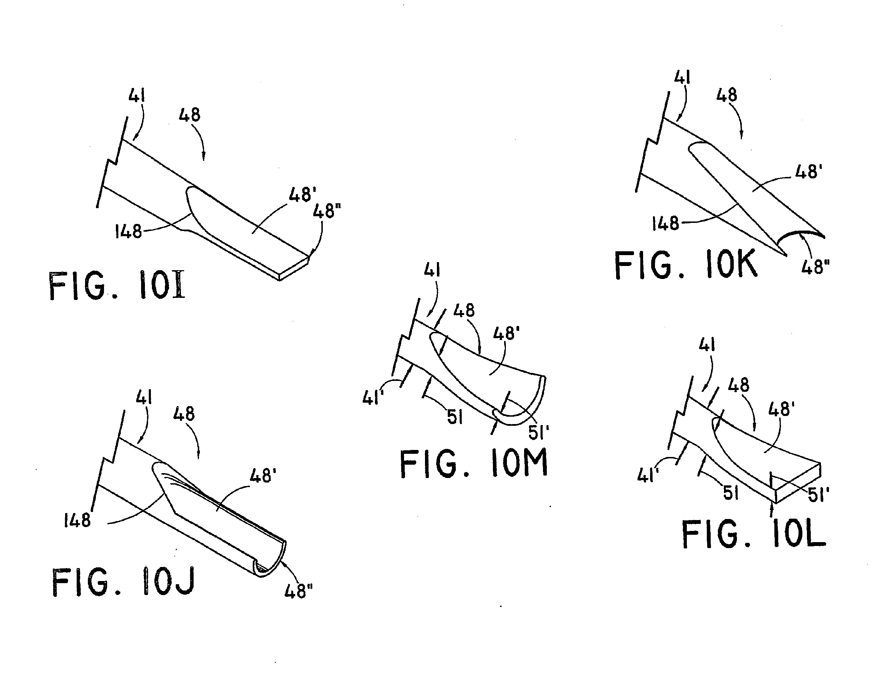

Turning to Figure 10, a schematic side view of an elongate inner compression member 41 according to an embodiment of the invention is shown, whereby like elements from the previous drawings, embodiments, and description from above are labeled the same. The inner compression member 41 may have the dimensions (diameter, length) a previously described. Similarly, the inner compression member 41 may comprise the materials as previously described. In one embodiment, the inner compression member 41 comprises a proximal end 40, a middle section 40', and a distal mating end 48 having an outer engaging surface 48'.

The overall length of the distal mating end 48 may vary. In one embodiment, it is from about 1.0 mm to about 10.0 mm. In another embodiment, the distal mating end 48 is approximately 3.0 mm. Furthermore, the distal mating end 48 optionally comprises a decreasing height 148 (Figs. 10, 10B) discussed below. The decreasing height 148 may extend along the length of the distal mating end 48 to the distal mating end outer engaging surface 48' and/or to a contoured configuration 48" that is described below. In one embodiment, the decreasing height 148 occurs along a length of about 3.0 mm, while in another embodiment the decreasing height 148 occurs over a much shorter length, such as approximately 0.5 mm. The contoured configuration 48" and outer engaging surface 48' may occur along the entire length of the distal mating end 48 or the decreasing height 148. Alternatively, the distal mating end 48 has a decreasing height 148 down to the contoured configuration 48" and outer engaging surface 48', and in one embodiment the decreasing height 148 is about 0.5 mm in length while the contoured configuration 48" and outer engaging surface 48' may extend distally from the decreasing height 148 and measure approximately 2.5 mm in length, for a distal mating end 48 that is approximately 3.0 mm in overall length.

Figures 10A and 10B show cross sectional views of Figure 10 taken along the lines 10A-10A and 10B-10B, whereby the inner compression member 41 in Figure 10A has an approximate height (e.g., outer diameter or thickness) of approximately 0.024 inches and Figure 10B shows an embodiment of a decreasing height 148 from less than about 0.24 inches to greater than about 0.08 inches, and in one embodiment the decreasing height 148 is approximately 0.016 inches from top 148' to bottom 148". The term "decreasing height" and variations thereof describe embodiments of the invention, rather than any lexicographic definition. For instance, the inner compression member distal mating end 48 may gradually decrease from top 148' to bottom 148" over the length of the mating end 48 in a variety of ways. In other words, the distal mating end 48 may have a portion that becomes gradually lesser in height (Fig. 10B)-relative to the inner compression member 41 (Fig. 10A)-in the distal direction. A decreasing height 148 may result from or be measured in a height, thickness, cross-section, diameter, width, and/or other configuration, shape, form, profile, structure, external outline, and/or contour that is lesser in height from the top 148', from the bottom 148", or as shown in Figure 10B from both the top 148' and the bottom 148". It should be understood that the top 148' and bottom 148" may be relative, and the inventions includes without limitation an embodiment where top 148' and bottom 148" are viewed end-on, a side view, or any view along the x-axis and y-axis from 0 to 2p (e.g., including the top 148' designated from 0 radians and bottom might be p radians). In one embodiment, the top 148' is the portion facing the outer engaging surface 164 of the base securing section 154.

While the decreasing height 148 may have a gradually smaller cross sectional area when compared to the cross sectional area at the inner compression member 41 (Figs. 10, 10A) that is proximal to the distal mating end 48, the cross sectional area need not decrease. For example, the width might increase inversely proportional to the decrease in height as shown in Figure 10B, although if desired the increased width could be cut away, machined, trimmed, chipped, planed, and the like. Also, this is not to say that the distal mating end 48 may only decrease in height. Indeed, the decreasing height 148 might extend to a position at or near the contoured configuration 48", which might increase in height, such as with an upward curve or other configuration that reverses to form a fluted embodiment, a flare, or upward protrusion. Furthermore, the distal mating end 48 may comprise more than one portion having a decreasing height, whereby the distal mating end 48 decreases in height from top 148' to bottom 148", then increases in height from top 148' to bottom 148", only to decrease again along the length of the distal mating end 48.

According to the invention, the inner compression member distal mating end 48 and/or outer engaging surface 48' further comprise a contoured configuration 48" that is generally complementary to the base securing section 162 and/or base securing section outer engaging surface 164. Thus, in one embodiment, the contoured configuration 48" and/or outer engaging surface 48' may have a boundary, periphery, or portion with a cross sectional shape that is fluted, whereby fluted comprises any curved, shoehorn shape, celery shape, semicircular shape, crescent shape, wishbone shape, saddle shape, C-shaped, V-shaped, U-shaped, or other arcuate configuration that curves radially inward or curves radially outward. In other embodiments, the contoured configuration 48" and/or outer engaging surface 48' may be substantially flat, whereby flat comprises having a horizontal surface without a slope, tilt, or curvature, alternatively having a substantially flat in a vertical plane, or alternatively having a tilt, angle, or taper comprising a one-sided beveled structure or working end of a screwdriver in the horizontal plane, vertical plane, or in between those planes, by way of example and not be way of limitation.

Figures 10C through 10H show, by way of example and not by way of limitation, various embodiments of a contoured configuration 48" and an outer engaging surface 48'. Figures 10A, 10B, 10C, 10D, 10E, 10F, 10G, and 10H show cross sectional views of Figure 10 taken along the lines 10A-10A, 10B-10B, 10C-10C, 10D-10D, 10E-10E, 10F-10F, 10G-10G, 10H-10H, respectively.

In Figures 10C and 10G, the contoured configuration 48" and outer engaging surface 48' are both fluted. While each figure shows a contoured configuration 48" that has decreased in height relative to the inner compression member 41 shown in the background with a circular cross section, the contoured configuration 48" in Figure 10G has an increased width.

In Figures 10E and 10F, the contoured configuration 48" and outer engaging surface 48' are both flat. While each figure shows a contoured configuration 48" that has decreased in height relative to the inner compression member 41 shown in the background with a circular cross section, the contoured configuration 48" in Figure 10F has an increased width.

In Figures 10D and 10H, the contoured configuration 48" is fluted and the outer engaging surface 48' is flat. While each figure shows a contoured configuration 48" that has decreased in height relative to the inner compression member 41 shown in the background with a circular cross section, the contoured configuration 48" in Figure 10H has an increased width.

Figures 10I, 10J, 10K, 10L, and 10M show perspective schematic views of alternative embodiments of an inner compression member distal mating end 48 according to the invention. Figure 10I shows that the distal mating end 48 has a decreasing height 148 down to a contoured configuration 48" and outer engaging surface 48' that are both flat. Thus, the decreasing height 148 in Figure 10I does not go to the distal tip of the distal mating end 48. The contoured configuration 48" and outer engaging surface 48' in Figure 10I may be achieved by swaging, stamping, forging, or rolling between two rollers.

Figure 10J shows that the distal mating end 48 has a decreasing height 148 down to a contoured configuration 48" and outer engaging surface 48' that are both fluted. Thus, the decreasing height 148 in Figure 10J does not go to the distal tip of the distal mating end 48. The contoured configuration 48" and outer engaging surface 48' in Figure 10J may be achieved by swaging, stamping, milling, and the like, or by rolling between two rollers followed by forging.

Figure 10K shows that the distal mating end 48 has a decreasing height 148 to its distal tip. Thus, a contoured configuration 48" and outer engaging surface 48' may be fluted, flat, or sloped along the length of the decreasing height 148. The contoured configuration 48" and outer engaging surface 48' in Figure 10K may be easily manufactured by grinding, machining, and the like.

Figure 10L shows that the distal mating end 48 has a decreasing height 148 down to a contoured configuration 48" and outer engaging surface 48' that are both flat. Thus, the decreasing height 148 in Figure 10I does not go to the distal tip of the distal mating end 48. While the contoured configuration 48" has decreased in height relative to the inner compression member 41 shown in the background, the contoured configuration 48" in Figure 10L has an increased width. The contoured configuration 48" and outer engaging surface 48' in Figure 10I may be achieved by swaging, stamping, forging, or rolling between two rollers.

Figure 10M shows that the distal mating end 48 has a decreasing height 148 down to a contoured configuration 48" and outer engaging surface 48' that are both fluted. Thus, the decreasing height 148 in Figure 10M does not go to the distal tip of the distal mating end 48. While the contoured configuration 48" has decreased in height relative to the inner compression member 41 shown in the background, the contoured configuration 48" in Figure 10M has an increased width. The contoured configuration 48" and outer engaging surface 48' in Figure 10M may be achieved by swaging, stamping, milling, and the like, or by rolling between two rollers followed by forging.

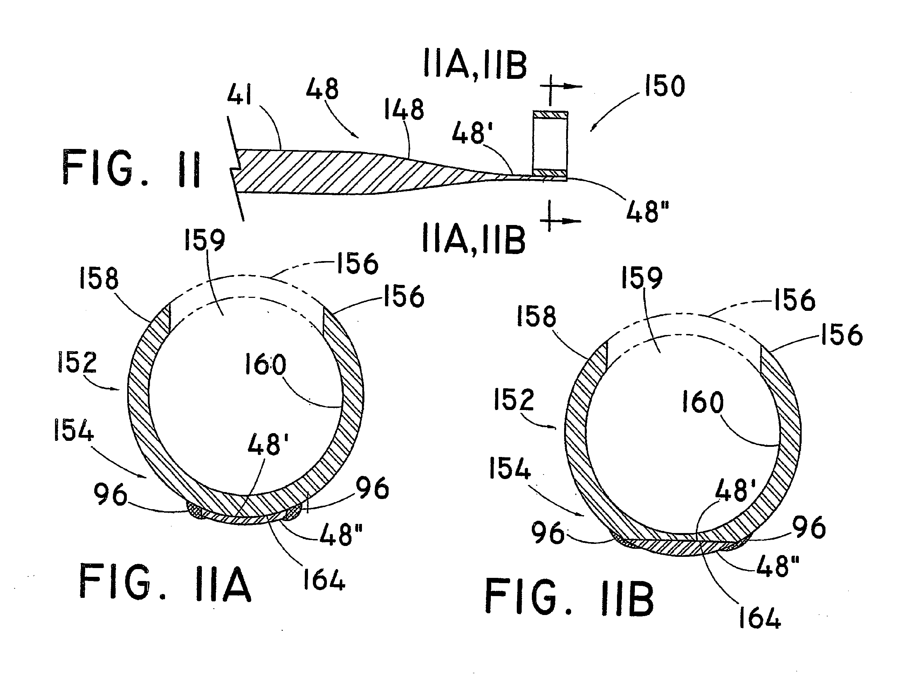

Figure 11 shows a longitudinal cross section of a securing body 150 operatively coupled to the distal mating end 48 of an inner compression member comprising a contoured configuration 48" and outer engaging surface 48'. Figures 11A and 11B show alternative embodiments of cross sectional views of Figure 11 taken along the lines 11A-11A and 11B-11B, respectively.

Figures 11A and 11B show, for instance, that the inner securing section 152 could be comprise the circumferential first end portion 156 of Figures 8A and 8B; Figures 11A and 11B show this using a reference numeral 156 having a dashed lead line that extends to a first end portion shown in phantom. Also, Figures 11 A and 11B show that the inner securing section 152 could comprise a first end portion 156 and a second end portion 158 of Figures 9A and 9B. As taught above, the inner securing portion 156 comprises a receiving cavity 159 and having inner engaging surface 160.

Figures 11A and 11B also show alternative embodiments of a base section 154 of the securing body 150, whereby Figure 11A shows a base securing section 154 having an outer engaging surface 164 in accordance with Figures 8A and 8B, while Figure 11B shows a base securing section 154 having an outer engaging surface 164 in accordance with Figures 9A and 9B. In Figures 11A and 11B, the inner compression member distal mating end outer engaging surface 48' and/or contoured configuration 48" is complementary to the base securing section outer engaging surface 164.

In addition, the base securing section 154 and the inner compression member distal mating end 48 are operatively coupled. In one embodiment, the outer engaging surface 48' of the mating end 48 is positioned in an abutting relationship (e.g., touching, in contact directly or by intervening parts, or adjacent) to the inner compression member distal mating end outer engaging surface 48' and/or contoured configuration 48 and then joined at a connection 96. The connection for operatively coupling may comprise any suitable means, including but not limiting to welding, soldering, brazing, or fusing. Thus, in one embodiment, the base securing section 154 and distal mating end 48 are operatively coupled by welding. In another embodiment, the base securing section 154 and distal mating end 48 are operatively coupled by soldering (including brazing and other similar techniques). In still another embodiment, the base securing section 154 and distal mating end 48 are operatively coupled by fusing the base securing section outer engaging surface 164 and the inner compression member distal mating end outer engaging surface 48'.

Soldering and brazing are used if a semi-permanent connection between the distal mating end 48 and the base securing section 154 is desired, because solder or braze metals have a lower melting point than the metals that are joined. Thus, when sufficient heat is applied to melt the solder or braze metal, they form an alloy with the surfaces of the mating end 48 and the base securing section 154 and, upon solidification, thus form a joint that can be unfastened during manufacturing (e.g., to redo in the event of a poor connection) by reheating without destroying the parts that have been joined. In contrast, welding involves melting the outer engaging surface 48' of the mating end 48 and an outer engaging surface of the base securing section 154 at the interface, or involves combining temperature and pressure so as to cause localized coalescence. Consequently, in most instances higher temperatures are involved than for soldering, and the union is permanent.

Although Figure 11 shows the distal most tip of the distal mating end 48 flush with (e.g., substantially co-planar) the distal end of the securing body 150, the distal most tip of the distal mating end 48 may alternatively be set back approximately 0.5 mm proximally from the distal end of the securing body 150. The set back arrangement allows solder, weld, or fusion to form a smooth transition and fill the space between that distal end tip and the securing body 150. This would also minimize the profile compared to placing more of a circumferential solder, weld, or fusion about the joint.

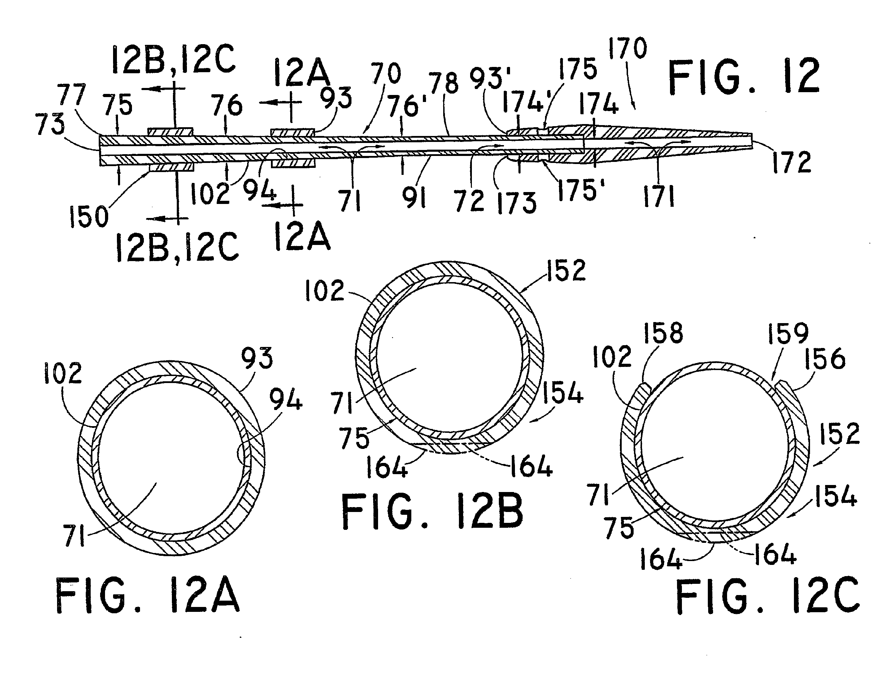

Turning to Figure 12, it shows an assembly comprising an inner guide channel member 70, atraumatic tip 170, proximal restraint 93, and securing body 150. The inner guide channel member has a distal first end 78 and a proximal second end 77, the second end 77 comprising an outer surface 102 and an outer diameter 75, a stent platform 91 disposed circumferentially about the inner guide channel member intermediate the first and second ends 78, 77, and entry and exit ports 72, 73, respectively, defining a guide channel 71 therebetween. In one embodiment, the outer diameter 75 is substantially uniform over the entire length of the inner guide channel member 70. Alternatively, the outer diameter 75 tapers 76 to a smaller second outer diameter 76' intermediate the outer guide channel member first and second ends 78, 77.

The atraumatic tip 170 has an entry port 172 and an exit port 173 defining an atraumatic tip guide channel 171 therebetween. The exit port 173 comprises a first bore inner diameter 174 that is distal to a larger second bore inner diameter 174'. The atraumatic tip 170 is operatively coupled to the inner guide channel member first end 78, which is received within the second bore inner diameter 174' and stops distally at the first bore inner diameter 174. At least a first glue port 175 extends from the outside surface of the atraumatic tip 170 to the atraumatic tip guide channel 171 for securing to the inner guide channel member first end 78. Optionally, a second glue port 175' extends from the outside surface of the atraumatic tip 170 to the atraumatic tip guide channel 171 for securing to the inner guide channel member first end 78. Any suitable glue that is biocompatible may be used to secure the atraumatic tip guide channel 171 and the inner guide channel member first end 78.

The atraumatic tip 170 may vary in length and outer diameter. In one embodiment, the atraumatic tip 170 is from about 5.0 mm to about 25.0 mm in length, while in another embodiment it measures approximately 17.0 mm in length. In one embodiment, the atraumatic tip 170 may be approximately 1.0 mm in outer diameter. In one embodiment, the first bore inner diameter 174 is approximately 0.0205 inches (or about 0.5207 mm) while the second bore inner diameter 174' is approximately 0.0290 inches (or about 0.5207 mm). In one embodiment, the first and second glue ports 175, 175', respectively, are located approximately 8.0 mm from the entry port 172 and approximately 8.0 mm from the exit port 173. These lengths, diameters, and other measurements are only illustrative of an embodiment of the atraumatic tip 170 according to the invention, and could vary as desired.

The proximal restraint 93 is sized to be large enough to stop the stent's proximal movement as previously explained. In one embodiment, the proximal restraint 93 is a restraining ring having a tubular configuration comprising an inner lumen surface 94 disposed circumferentially about the inner guide channel member 70. The tubular configuration may comprise a cylinder that is circular, right cylindrical, elliptical, hyperbolic, parabolic, curved, polygonal, rectangular, or irregular in shape or cross section. Also, the restraining ring may have a slit in its wall before or after the inner lumen surface 94 is disposed circumferentially about the inner guide channel member 70.

In one embodiment, the restraining ring's length is less than its greatest outer diameter. In yet another embodiment, the restraining ring is elongate in that its length is greater than its outer diameter. For example, the restraining ring may have an inner diameter of approximately 0.076 cm (0.030 inches), an outer diameter of approximately 0.129 cm (0.051 inches), and a length of approximately 3.0 mm. The restraining ring inner diameter 0.076 cm (0.030 inches) is disposed about an inner guide channel member outer diameter that is from about 0.04 cm (0.016 inches) to about 0.06 cm (0.024 inches), whereby glue that is used to operatively couple the inner lumen surface 94 to the inner guide channel member 70 will fill the differential between the restraining ring inner diameter and the inner guide channel member outer diameter.

Figures 12A, 12B, and 12C show cross sectional views of Figure 12 taken along the lines 12A-12A, 12B-12B, and 12C-12C. Figure 12A shows a cross section of the proximal restraint 93 such as a restraining ring described above, the restraining ring inner lumen surface 94 disposed circumferentially about and glued (not shown) to an inner guide channel member outer surface 102, leaving the guide channel 71 of the inner guide channel member 70 unobstructed.

Figures 12B and 12C show cross sections of alternative embodiments of a securing body 150 according to the invention being disposed circumferentially about the inner guide channel member outer surface 102 and operatively coupled as described above. Specifically, Figure 12B comprises an inner securing section 152 having a first end portion 156 shown in Figures 8A and 8B, while Figure 12C comprises an inner securing section 152 having a first end portion 156 and a second end portion 158 of Figures 9A and 9B shown in Figures 9A and 9B.

Figures 12A and 12B show alternative embodiments of the base securing section 154. The base securing section 154 according to Figures 8A and 8B is shown using a reference numeral having a solid lead line for the outer engaging surface 164. The base securing section 154 according to Figures 9A and 9B is shown using a dashed lead line that extends to a phantom portion that has been removed for the outer engaging surface 164.

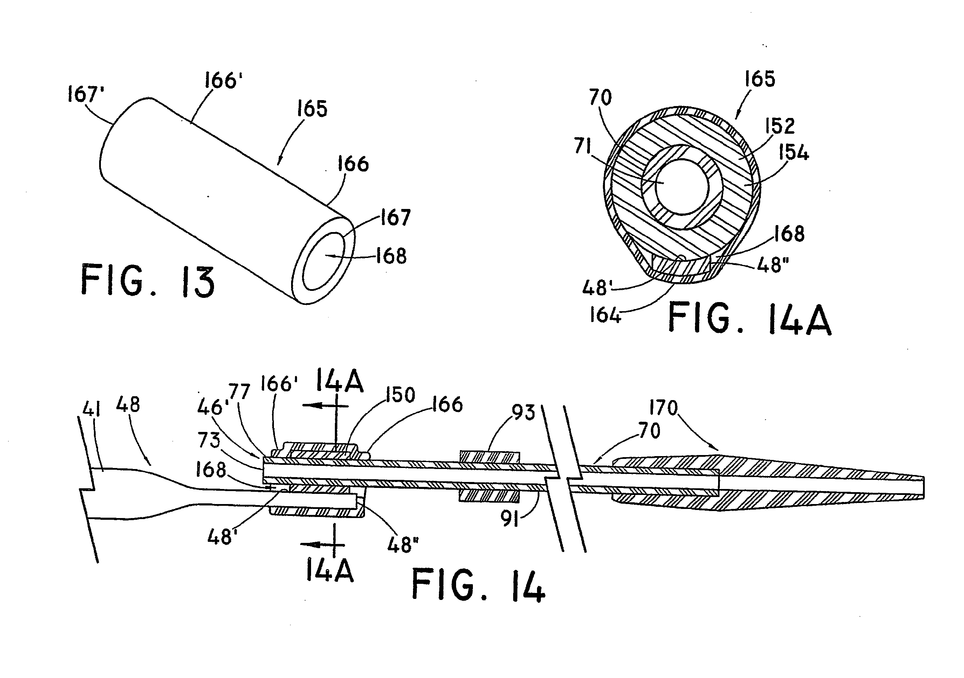

Figure 13 shows an optional tube 165 comprising a first end 166 and a second end 166'. The first end 166 has a first end opening 167 and the second end 166' has a second end opening 167'. The first and second end openings 167, 167', respectively, define a lumen 168 therebetween. Where the inner compression member distal mating end 48 and the base securing section 154 are operatively coupled by welding, soldering, brazing, or fusing, the tube 165 may be disposed about the joint. The tubing 165 has the advantage of minimizing some of the sharp edges created by a welded, soldered, or fused joint.

Figure 14 shows a joint assembly 46' comprising an inner compression member 41 having a distal mating end 48 operatively coupled to a securing body 150, which securing body 150 operatively couples a second end 77 of an inner guide channel member 70. Figure 14 also shows the inner guide channel member 70 having an atraumatic tip 170 operatively coupled to the first end 78 of the inner guide channel member 70, wherein a proximal restraint 93 such as a restraining ring is circumferentially disposed distal to the securing body 150 and proximal to the atraumatic tip 170. An outer stent platform 91 is disposed about the inner guide channel member 70 intermediate the first and second ends 78, 77, respectively, of the inner guide channel member 70.

The optional tube 165 disposes about the joint assembly 46'. The lumen 168 of the tube 165 optionally is sufficiently large to be slideable over the joint assembly (e.g., the region where the inner securing inner securing section 152 of the securing body 150 operatively couples the inner guide channel member 70 and the base securing section 154 the of the securing body 150 operatively couples the outer engaging surface 48' and/or contoured configuration 48" of the distal mating end 48 of the inner compression member 41). In the alternative, the tube 165 may be a shrink tubing (inclusive of shrink material that can be wrapped around the joint assembly 46').

As shown in Figure 14 and Figure 14A, a cross section taken along the lines 14A-14A of Figure 14, the tube 165 comprises a shrink tubing disposed about the joint assembly 46'. Heat shrink tubing, available from many suppliers, including Zeus, Inc. in Orangeburg, South Carolina for instance and also Cobalt Polymers in Cloverdale, California, may be disposed about the joint assembly 46. Heating the tube 165 causes it to melt about the joint assembly 46'. Upon cooling, a solid-state bond results between the tube 165 and the securing body 150, the inner guide channel member 70, and the inner compression member distal mating end 48. METHOD

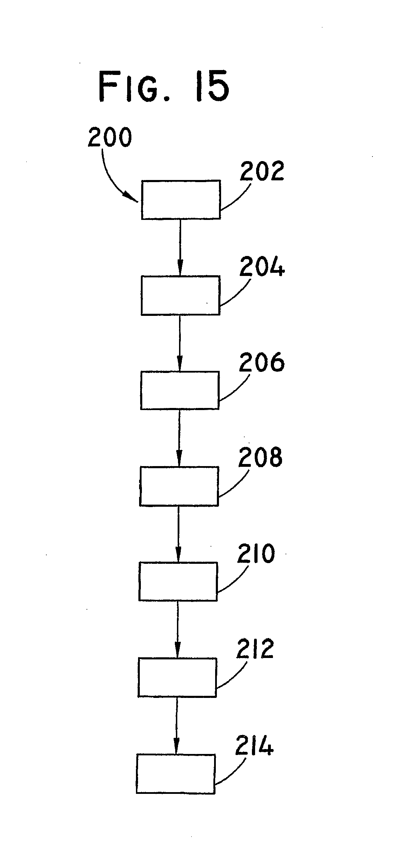

Methods of manufacturing an internal joint for use in a medical device delivery system for deploying an implantable prosthesis (e.g., a self-expanding, balloon expandable, or non-expanding stent; prosthetic valve devices, and other implantable articles) at a selected location inside a patient's body are also provided. Figure 15 is a block diagram illustrating a method 200 of manufacturing an internal joint for use in a medical device delivery system according to the present invention.

In step 202, an inner guide channel member 70 is provided having a distal first end 78 with an entry port 72 and a proximal second end 77 with an exit port 73, the entry and exit ports 72, 73, respectively, defining a guide channel 71 therebetween. The inner guide channel member second end 77 comprises an outer diameter 75 and an outer surface 102.

In step 204, a securing body 150 is provided. The securing body 150 comprises an inner securing section 152 and a base securing section 154, the inner securing section 152 having a first end portion 156 and a second end portion 158 and defining a receiving cavity 159 therebetween. The receiving cavity 159 is sized to receive the inner guide channel member second end outer diameter 75 and has at least one inner engaging surface 160. The base securing section 154 comprises a curved portion 162 and an outer engaging surface 164.

In step 206, an elongate inner compression member 41 is provided. The inner compression member 41 comprises a proximal end 40 and a distal mating end 48, and an optional middle section 40'.

In step 208, the inner compression member distal mating end 48 is formed comprising an engaging surface 48' and a contoured configuration 48" complementary to the base securing section outer engaging surface 164. In order to create the contoured configuration 48", the inner compression member distal mating end 48 may be formed, sheared, casted, or molded. By way of example only, forming can be done both hot and cold (except for stamping, which is always done cold) in order to modify the shape and/or physical properties of the material comprising the inner compression member distal mating end 48. Common forming processes include rolling the distal mating end 48 (between one or two rollers), stretching, forging, straight bending, and stamping.

In one embodiment, forming (step 208) the contoured configuration 48" comprises the step of rolling the inner compression member distal mating end 48 from a round to a fluted configuration complementary to the base securing section outer engaging surface 164. In another embodiment, forming (step 208) the contoured configuration 48" comprises the step of forging the inner compression member distal mating end 48 from a round to a fluted configuration complementary to the base securing section outer engaging surface 164. In another embodiment, forming (step 208) the contoured configuration 48" comprises the step of rolling the inner compression member distal mating end 48 from a round to a flat configuration complementary to the base securing section outer engaging surface 164. In another embodiment, forming (step 208) the contoured configuration 48" comprises the step of forging the inner compression member distal mating end 48 from a round to a flat configuration complementary to the base securing section outer engaging surface 164.

In step 210, the base securing section outer engaging surface 164 and the distal mating end engaging surface 48' disposed in an abutting relationship (e.g., touching, in contact directly or by intervening parts, or adjacent).

In step 212, the inner guide channel member second end 77 is inserted within the securing body inner securing section receiving cavity 159. The receiving cavity inner engaging surface 160 and the inner guide channel member second end outer surface 102 are operatively coupled directly or indirectly by a mechanically, chemically, and/or chemical-mechanically bond. In one embodiment, the bond may comprise a friction fit, press fit tight, nesting, wedging, crimping, clamping, pinchers, teeth, barbs, or any combination thereof, to name a few. In another embodiment, the bond may comprise glue, adhesives, resins, chemical bonding materials or combinations thereof and the like between the base securing section outer engaging surface 160 and the inner guide channel member second end outer surface 102.

In step 214, the base securing section 154 and distal mating end 48 are operatively coupled. In one embodiment, they are operatively coupled by welding. In another embodiment, they are operatively coupled by soldering, which includes soldering and brazing. In another embodiment, they are operatively coupled by fusing, which includes but is not limited to glue, adhesives, resins, chemical bonding materials, or combinations thereof.

A method of manufacturing the internal joint need not be performed sequentially. By way of example, the securing body 150 may be provided (step 204) before the inner compression member 41 (step 206), which may be provided before the inner guide channel member 70 is provided (step 202). Likewise, the inner guide channel member second end may be inserted (step 212) and operatively coupled within the securing body inner securing section receiving cavity 159 before the base securing section outer engaging surface 164 is disposed (step 210) in an abutting relationship relative to the distal mating end engaging surface 48'.

It is intended that the foregoing detailed description of a joint assembly for operatively coupling an inner compression member and an inner guide channel member a medical device having a tapered inner guide channel member for use with medical device delivery systems and medical devices, and methods of forming the joint assembly, and inner joint with a tapered inner compression member be regarded as illustrative rather than limiting, and that it be understood that it is the following claims, including all equivalents, that are intended to define the spirit and scope of this invention. Terms are to be given their reasonable plain and ordinary meaning. Also, the embodiment of any figure and features thereof may be combined with the embodiments depicted in other figures. Other features known in the art and not inconsistent with the structure and function of the present invention may be added to the embodiments.

While particular elements, embodiments, and applications of the present invention have been shown and described, it will be understood, of course, that the invention is not limited thereto since modifications may be made by those skilled in the art, particularly in light of the foregoing teachings. Therefore, it is therefore contemplated by the appended claims to cover such modifications as incorporate those features which come within the scope of the invention.

## Claims

1. An internal joint for use in a medical device delivery system for deploying an implantable prosthesis at a selected location inside a patient's body, comprising:
an inner guide channel member (70) having a distal first end (78) and a proximal second end (77), the second end (77) comprising an outer diameter (75) and an outer surface (102);
a securing body (150) defining a receiving cavity (159) that is sized to receive the inner guide channel member second end outer diameter (75), the receiving cavity (159) having at least one inner engaging surface (160) disposed about and operatively coupled to the inner guide channel member second end outer surface (102), and the securing body (150) comprising an outer engaging surface (164); and
an elongate inner compression member (41) having a proximal end (40) and a distal mating end (48), the distal mating end comprising an engaging surface (48') and a contoured configuration (48") complementary to the securing body outer engaging surface (164),
wherein the outer engaging surface (164) and the distal mating end engaging surface (48') are abutted, and the securing body (150) and distal mating end (48) are operatively coupled.

2. The internal joint of claim 1 wherein the securing body (150) comprises metal.

3. The internal joint of claim 1 or 2 wherein the securing body (150) comprises a generally tubular cannula (95).

4. The internal joint of claim 1 wherein the inner compression member distal mating end contoured configuration (48") is fluted and complementary to the securing body outer engaging surface (164) that is curved.

5. The internal joint of claim 1 wherein the inner compression member distal mating end contoured configuration (48") is flat and complimentary to the securing body outer engaging surface (164) that is flat.

6. The internal joint of claim 1 wherein the securing body (150) is generally ring-shaped.

7. The internal joint of claim 1 wherein the securing body (150) is generally U-shaped.

8. The internal joint of claim 1 wherein the securing body (150) and distal mating end (48) are operatively coupled by welding or soldering.

9. The internal joint of claim 1 wherein the securing body (150) and distal mating end (48) are operatively coupled by fusing the securing body outer engaging surface (164) and the distal mating end engaging surface (48').

10. The internal joint of claim 1 wherein the receiving cavity inner engaging surface (160) and the inner guide channel member second end outer engaging surface (102) are operatively coupled by a bond selected from the group consisting of a crimp, friction fit, press fit, wedge, glue, adhesive, resin, welding, chemical bonding materials, and combinations thereof.

11. The internal joint of claim 1 wherein the inner guide channel member first end (78) comprises an entry port (72) and the inner guide channel member second end (77) comprises an exit port (73), the entry port (72) and exit port (73) define a guide channel (71) therebetween.

12. The internal joint of claim 11 wherein the inner guide channel member (70) further comprises an outer stent platform (91) intermediate the first and second ends (78, 77) of the inner guide channel member ends and a proximal stent restraint (93).

13. The internal joint of claim 11 wherein the inner guide channel member (70) further comprises a taper (76) intermediate the first and second ends (78, 77) of the inner guide channel member ends, the taper (76) having a distal second outer diameter (76') less than said second end outer diameter (75).

14. A medical device delivery system for deploying an implantable prosthesis at a selected location inside a patient's body, the delivery system comprising the internal joint of any one of the preceding claims, wherein said device further comprises:
a stent platform (91) disposed circumferentially about the inner guide channel member (70) intermediate the first and second ends (78, 77);
an atraumatic tip (170) operatively coupled to the inner guide channel member first end (78), the atraumatic tip comprising a distal stent restraint (93'); and
a restraining ring disposed about and operatively coupled to the inner guide channel member (70) intermediate the inner guide channel member first and second ends (78, 77), the restraining ring comprising a proximal stent restraint (93).

15. The device of claim 14 wherein the securing body (150) and distal mating end (48) of the inner compression member (41) are operatively coupled by a bond selected from the group consisting of a welding, soldering, gluing, fusing, and combinations thereof.

16. The device of claim 15 further comprising a shrink tubing (165) disposed about the bond and a portion of the securing body (150).

17. The device of claim 14 wherein the atraumatic tip (170) further comprises a distal first end (178) with an entry port (172) and a proximal second end (177) with an exit port (173) and defining a guide channel (171), the atraumatic tip second end (177) comprising said distal stent restraint (93') and being operatively coupled to the inner guide channel member first end (78) such that the atraumatic tip guide channel (171) is in fluid communication with the guide channel (71) of the inner guide channel member (70).

18. The device of claim 14 further comprising a rapid insertion catheter delivery system, the system comprising an elongate outer sheath (50) having a proximal end (57) and a distal end (58) defining a passageway (59) therebetween that slideably receives a portion of the inner compression member (41), the outer sheath distal end (58) being operatively coupled to an outer guide channel member (80) having first and second ends (88, 87) defining a guide channel (81) that slideably receives the inner guide channel member (70), a breech position opening (65) at or near the outer guide channel member second end (87) and in fluid communication with both guide channels (71, 81), and a handle (30) operatively coupled to the outer sheath proximal end (57) and axially slideable relative to a stylet (20) operatively coupled to the inner compression member proximal end (40) and intended to remain outside said patient.

19. A method of making an internal joint for use in a medical device delivery system for deploying an implantable prosthesis at a selected location inside a patient's body, comprising the steps of:
providing an inner guide channel member (70) having a distal first end (78) with an entry port (72) and a proximal second end (77) with an exit port (73), the entry and exit ports (72, 73) defining a guide channel (71) therebetween, the second end (77) comprising an outer diameter (75) and an outer surface (102);
providing a securing body (150) defining a receiving cavity (159) that is sized to receive the inner guide channel member second end outer diameter (75), the receiving cavity (159) having at least one inner engaging surface (160), and the securing body (150) comprising a curved portion and an outer engaging surface (164);
providing an elongate inner compression member (41) having a proximal end (40) and a distal mating end (48);
forming the inner compression member distal mating end (48) in a form comprising an engaging surface (48') and a contoured configuration (48") complementary to the securing body outer engaging surface (164);
disposing the securing body outer engaging surface (164) in abutting relationship to the distal mating end engaging surface (48');
inserting the inner guide channel member second end (77) within the securing body receiving cavity (159);
operatively coupling the receiving cavity inner engaging surface (160) and the inner guide channel member second end outer surface (102).

20. The method of claim 19 further comprising the step of welding the securing body (150) and distal mating end (48).

21. The method of claim 19 further comprising the step of soldering the securing body (150) and distal mating end (48).

22. The method of claim 19 further comprising the step of fusing the securing body outer engaging surface (164) and the distal mating end engaging surface (48').

23. The method of claim 19 wherein the operatively coupling of the receiving cavity inner engaging surface (160) and the inner guide channel member second end outer surface (102) comprises the step of forming a bond selected from the group consisting of a crimp, friction fit, press fit, wedge, glue, adhesive, resin, welding, chemical bonding materials, and combinations thereof.

24. The method of claim 19 wherein the step of forming the contoured configuration (48") comprises the step of rolling or forging the inner compression member distal mating end (48) from a round to a fluted configuration complementary to the securing body outer engaging surface (164).

25. The method of claim 19 wherein the step of forming the contoured configuration (48") comprises the step of rolling or forging the inner compression member distal mating end (48) from a round to a flat configuration complementary to the securing body outer engaging surface (164).

26. The method of claim 23 wherein the forming of the contoured configuration (48") comprises the step of forging the inner compression member distal mating end (48) from a round to a flat configuration complementary to the securing body outer engaging surface (164).

## Patentansprüche

1. Innengelenk zur Verwendung in einem System zur Zuführung von medizinischen Geräten zum Einsetzen einer implantierbaren Prothese an einer ausgewählten Stelle im Körper eines Patienten; umfassend
ein inneres Führungskanalelement (70) mit einem distalen ersten Ende (78) und einem proximalen zweiten Ende (77), wobei das zweite Ende (77) einen Außendurchmesser (75) und eine Außenfläche (102) umfasst;
einen Fixierungskörper (150), der einen Aufnahmehohlraum (159) definiert, der eine Größe zur Aufnahme des Außendurchmessers (75) des zweiten Endes des inneren Führungskanalelements aufweist, wobei der Aufnahmehohlraum (159) zumindest eine innere Eingriffsfläche (160) aufweist, die um die Außenfläche (102) des zweiten Endes des inneren Führungskanalelements angeordnet ist und damit in Wirkverbindung steht, und wobei der Fixierungskörper (150) eine äußere Eingriffsfläche (164) umfasst; und ein langgestrecktes inneres Kompressionselement (41) mit einem proximalen Ende (40) und einem distalen passenden Ende (48), wobei das distale passende Ende eine Eingriffsfläche (48') und eine konturierte Konfiguration (48") umfasst, die zu der äußeren Eingriffsfläche (164) des Fixierungskörpers komplementär ist,
worin die äußere Eingriffsfläche (164) und die Eingriffsfläche (48') des distalen passenden Endes aneinander stoßen, und der Fixierungskörper (150) und das distale passende Ende (48) in Wirkverbindung stehen.

2. Innengelenk nach Anspruch 1, worin der Fixierungskörper (150) Metall umfasst.

3. Innengelenk nach Anspruch 1 oder 2, worin der Fixierungskörper (150) eine allgemein röhrenförmige Kanüle (95) umfasst.

4. Innengelenk nach Anspruch 1, worin die konturierte Konfiguration (48") des distalen passenden Endes des inneren Kompressionselements gerillt ist und zu der gebogenen äußeren Eingriffsfläche (164) des Fixierungskörpers komplementär ist.

5. Innengelenk nach Anspruch 1, worin die konturierte Konfiguration (48") des distalen passenden Endes des inneren Kompressionselements flach ist und zu der flachen äußeren Eingriffsfläche (164) des Fixierungskörpers komplementär ist.

6. Innengelenk nach Anspruch 1, worin der Fixierungskörper (150) allgemein ringförmig ist.

7. Innengelenk nach Anspruch 1, worin der Fixierungskörper (150) allgemein U-förmig ist.

8. Innengelenk nach Anspruch 1, worin der Fixierungskörper (150) und das distale passende Ende (48) durch Schweißen oder Löten in Wirkverbindung gebracht werden.

9. Innengelenk nach Anspruch 1, worin der Fixierungskörper (150) und das distale passende Ende (48) in Wirkverbindung gebracht werden, indem die äußere Eingriffsfläche (164) des Fixierungskörpers und die Eingriffsfläche (48') des distalen passenden Endes miteinander verschmolzen werden.

10. Innengelenk nach Anspruch 1, worin die innere Eingriffsfläche (160) des Aufnahmehohlraums und die äußere Eingriffsfläche (102) des zweiten Endes des inneren Führungskanalelements durch eine Verbindung miteinander in Wirkverbindung stehen, die ausgewählt ist aus der aus Klemmverbindung, reibschlüssige Passung, Presspassung, Keil, Klebstoff, Klebemittel, Harz, Schweißen, chemische Verbindungsmittel und Kombinationen daraus bestehenden Gruppe.

11. Innengelenk nach Anspruch 1, worin das erste Ende (78) des inneren Führungskanalelements eine Eingangsöffnung (72) umfasst und das zweite Ende (77) des inneren Führungskanalelements eine Ausgangsöffnung (73) umfasst, wobei die Eingangsöffnung (72) und die Ausgangsöffnung (73) einen Führungskanal (71) dazwischen definieren.

12. Innengelenk nach Anspruch 11, worin das innere Führungskanalelement (70) ferner eine äußere Stentplattform (91) zwischen den ersten und zweiten Enden (78, 77) der Enden des inneren Führungskanalelements und eine proximale Stenthalterung (93) umfasst.

13. Innengelenk nach Anspruch 11, worin das innere Führungskanalelement (70) ferner eine Verjüngung (76) zwischen den ersten und zweiten Enden (78, 77) der Enden des inneren Führungskanalelements umfasst, wobei die Verjüngung (76) einen distalen zweiten Außendurchmesser (76') aufweist, der kleiner ist als der Außendurchmesser (75) des zweiten Endes.

14. System zur Zuführung von medizinischen Geräten zum Einsetzen einer implantierbaren Prothese an einer ausgewählten Stelle im Körper eines Patienten, wobei das Zuführungssystem das Innengelenk nach einem der vorhergehenden Ansprüche umfasst, worin die Vorrichtung ferner Folgendes umfasst:
eine Stentplattform (91), die um den Umfang des inneren Führungskanalelements (70) zwischen den ersten und zweiten Enden (78, 77) angeordnet ist,
eine atraumatische Spitze (170), die mit dem ersten Ende (78) des inneren Führungskanalelements in Wirkverbindung steht, wobei die atraumatische Spitze eine distale Stenthalterung (93') umfasst; und
einen Haltering, der um das innere Führungskanalelement (70) herum zwischen den ersten und zweiten Enden (78, 77) des inneren Führungskanalelements angeordnet ist und damit in Wirkverbindung steht, wobei der Haltering eine proximale Stenthalterung (93) umfasst.

15. Vorrichtung nach Anspruch 14, worin der Fixierungskörper (150) und das distale passende Ende (48) des inneren Kompressionselements (41) durch eine Verbindung in Wirkverbindung stehen, die aus der aus Schweißverbindung, Lötverbindung, Klebeverbindung, Schmelzverbindung und Kombinationen davon bestehenden Gruppe ausgewählt ist.

16. Vorrichtung nach Anspruch 15, ferner umfassend einen Schrumpfschlauch (165) der um die Verbindung und einen Teil des Fixierungskörpers (150) angeordnet ist.

17. Vorrichtung nach Anspruch 14, worin die atraumatische Spitze (170) ferner ein distales erstes Ende (178) mit einer Eingangsöffnung (172) und ein proximales zweites Ende (177) mit einer Ausgangsöffnung (173) umfasst, die einen Führungskanal (171) definiert, wobei das zweite Ende (177) der atraumatischen Spitze die distale Stenthalterung (93') umfasst und mit dem ersten Ende (78) des inneren Führungskanalelements in Wirkverbindung steht, so dass der Führungskanal (171) der atraumatischen Spitze mit dem Führungskanal (71) des inneren Führungskanalelements (70) in Fluidverbindung steht.

18. Vorrichtung nach Anspruch 14, ferner umfassend ein schnell einführbares Katheterzuführungssystem, wobei das System eine langgestreckte Außenschleuse (50) mit einem proximalen Ende (57) und einem distalen Ende (58) umfasst, die einen Durchgang (59) zwischen sich definieren, der einen Teil des inneren Kompressionselements (41) gleitend aufnimmt, wobei das distale Ende (58) der Außenschleuse mit einem äußeren Führungskanalelement (80) mit ersten und zweiten Enden (88, 87) in Wirkverbindung steht, die einen Führungskanal (81) definieren, der das innere Führungskanalelement (70) gleitend aufnimmt, eine Endlagenöffnung (65) an dem zweiten Ende (87) des äußeren Führungskanalelements oder in dessen Nähe und in Fluidverbindung mit beiden Führungskanälen (71, 81), und einen Handgriff (30), der mit dem proximalen Ende (57) der Außenschleuse in Wirkverbindung steht und bezüglich eines Mandrins (20) axial gleiten kann, der mit dem proximalen Ende (40) des inneren Kompressionselements in Wirkverbindung steht und außerhalb des Patienten bleiben soll.

19. Verfahren zur Herstellung eines Innengelenks zur Verwendung in einem System zur Zuführung von medizinischen Geräten zum Einsetzen einer implantierbaren Prothese an einer ausgewählten Stelle im Körper eines Patienten, das folgende Schritte umfasst:
Bereitstellung eines inneren Führungskanalelements (70) mit einem distalen ersten Ende (78) mit einer Eingangsöffnung (72) und einem proximalen zweiten Ende (77) mit einer Ausgangsöffnung (73), wobei die Eingangs- und Ausgangsöffnungen (72, 73) einen Führungskanal (71) dazwischen definieren, wobei das zweite Ende (77) einen Außendurchmesser (75) und eine Außenfläche (102) umfasst;
Bereitstellung eines Fixierungskörpers (150), der einen Aufnahmehohlraum (159) definiert, der eine Größe zur Aufnahme des Außendurchmessers (75) des zweiten Endes des inneren Führungskanalelements aufweist, wobei der Aufnahmehohlraum (159) zumindest eine innere Eingriffsfläche (160) aufweist und wobei der Fixierungskörper (150) einen gebogenen Abschnitt und eine äußere Eingriffsfläche (164) umfasst;
Bereitstellung eines langgestreckten inneren Kompressionselements (41) mit einem proximalen Ende (40) und einem distalen passenden Ende (48);
Formen des distalen passenden Endes (48) des inneren Kompressionselements in einer Form, die eine Eingriffsfläche (48') und eine konturierte Konfiguration (48") umfasst, die zu der äußeren Eingriffsfläche (164) des Fixierungskörpers komplementär ist;
Anordnung der äußeren Eingriffsfläche (164) des Fixierungskörpers in anstoßender Beziehung zu der Eingriffsfläche (48') des distalen passenden Endes;
Einführung des zweiten Endes (77) des inneren Führungskanalelements in den Aufnahmehohlraum (159) des Fixierungskörpers;
in Wirkverbindung bringen der inneren Eingriffsfläche (160) des Aufnahmehohlraums mit der Außenfläche (102) des zweiten Endes des inneren Führungskanalelements.

20. Verfahren nach Anspruch 19, ferner umfassend den Schritt der Schweißverbindung des Fixierungskörpers (150) und des distalen passenden Endes (48).

21. Verfahren nach Anspruch 19, ferner umfassend den Schritt der Lötverbindung des Fixierungskörpers (150)und des distalen passenden Endes (48).

22. Verfahren nach Anspruch 19, ferner umfassend den Schritt der Schmelzverbindung der äußeren Eingriffsfläche (164) des Fixierungskörpers und der Eingriffsfläche (48') des distalen passenden Endes.

23. Verfahren nach Anspruch 19, worin das in Wirkverbindung bringen der inneren Eingriffsfläche (160) des Aufnahmehohlraums und der Außenfläche (102) des zweiten Endes des inneren Führungskanalelements den Schritt des Formens einer Verbindung umfasst, die aus der aus Klemmverbindung, reibschlüssige Passung, Presspassung, Keil, Klebstoff, Klebemittel, Harz, Schweißen, chemische Verbindungsmittel und Kombinationen daraus bestehenden Gruppe ausgewählt ist.

24. Verfahren nach Anspruch 19, worin der Schritt des Formens der konturierten Konfiguration (48") den Schritt des Rollens oder Schmiedens des distalen passenden Endes (48) des inneren Kompressionselements von einer runden zu einer ausgekehlten Konfiguration, die zu der äußeren Eingriffsfläche (164) des Fixierungskörpers komplementär ist, umfasst.

25. Verfahren nach Anspruch 19, worin der Schritt des Formens der konturierten Konfiguration (48") den Schritt des Rollens oder Schmiedens des distalen passenden Endes (48) des inneren Kompressionselements von einer runden zu einer flachen Konfiguration, die zu der äußeren Eingriffsfläche (164) des Fixierungskörpers komplementär ist, umfasst.

26. Verfahren nach Anspruch 23, worin das Formen der konturierten Konfiguration (48") den Schritt des Schmiedens des distalen passenden Endes (48) des inneren Kompressionselements von einer runden zu einer flachen Konfiguration, die zu der äußeren Eingriffsfläche (164) des Fixierungskörpers komplementär ist, umfasst.

## Revendications

1. Joint interne pour l'utilisation dans un système d'introduction de dispositif médical destiné à déployer une prothèse implantable au niveau d'un emplacement sélectionné à l'intérieur du corps d'un patient, comprenant :
un organe de canal de guidage interne (70) ayant une première extrémité distale (78) et une deuxième extrémité proximale (77), la deuxième extrémité (77) comprenant un diamètre extérieur (75) et une surface externe (102) ;
un corps de fixation (150) définissant une cavité de réception (159) dimensionnée pour recevoir le diamètre extérieur (75) de la deuxième extrémité de l'organe de canal de guidage interne, la cavité de réception (159) ayant au moins une surface d'engagement interne (160) disposée autour de, et
accouplée fonctionnellement à, la surface externe (102) de la deuxième extrémité de l'organe de canal de guidage interne, et le corps de fixation (150) comprenant une surface d'engagement externe (164) ; et
un organe de compression interne allongé (41) ayant une extrémité proximale (40) et une extrémité d'accouplement distale (48), l'extrémité d'accouplement distale comprenant une surface d'engagement (48') et une configuration profilée (48 ") complémentaire de la surface d'engagement externe (164) du corps de fixation,
la surface d'engagement externe (164) et la surface d'engagement (48') de l'extrémité d'accouplement distale étant en aboutement, et le corps de fixation (150) et l'extrémité d'accouplement distale (48) étant accouplés fonctionnellement.

2. Joint interne selon la revendication 1, dans lequel le corps de fixation (150) comprend du métal.

3. Joint interne selon la revendication 1 ou 2, dans lequel le corps de fixation (150) comprend une canule généralement tubulaire (95).

4. Joint interne selon la revendication 1, dans lequel la configuration profilée (48") de l'extrémité d'accouplement distale de l'organe de compression interne est cannelée et est complémentaire à la surface d'engagement externe (164) du corps de fixation, qui est courbe.

5. Joint interne selon la revendication 1, dans lequel la configuration profilée (48") de l'extrémité d'accouplement distale de l'organe de compression interne est plate et est complémentaire à la surface d'engagement externe (164) du corps de fixation, qui est plate.

6. Joint interne selon la revendication 1, dans lequel le corps de fixation (150) est généralement de forme annulaire.

7. Joint interne selon la revendication 1, dans lequel le corps de fixation (150) est généralement en forme de U.

8. Joint interne selon la revendication 1, dans lequel le corps de fixation (150) et l'extrémité d'accouplement distale (48) sont accouplés fonctionnellement par soudage ou brasage.

9. Joint interne selon la revendication 1, dans lequel le corps de fixation (150) et l'extrémité d'accouplement distale (48) sont accouplés fonctionnellement par fusion de la surface d'engagement externe (164) du corps de fixation et de la surface d'engagement (48') de l'extrémité d'accouplement distale.

10. Joint interne selon la revendication 1, dans lequel la surface d'engagement interne (160) de la cavité de réception et la surface d'engagement externe (102) de la deuxième extrémité de l'organe de canal de guidage interne sont accouplées fonctionnellement par une liaison choisie parmi le groupe constitué d'un sertissage, d'un ajustement par friction, d'un ajustement par pressage, d'une cale, d'une colle, d'un adhésif, d'une résine, d'une soudure, de matériaux de liaison chimique et de combinaisons de ceux-ci.

11. Joint interne selon la revendication 1, dans lequel la première extrémité (78) de l'organe de canal de guidage interne comprend un orifice d'entrée (72) et la deuxième extrémité (77) de l'organe de canal de guidage interne comprend un orifice de sortie (73), l'orifice d'entrée (72) et l'orifice de sortie (73) définissant entre eux un canal de guidage (71).

12. Joint interne selon la revendication 11, dans lequel l'organe de canal de guidage interne (70) comprend en outre une plate-forme d'endoprothèse externe (91) entre la première et la deuxième extrémité (78, 77) des extrémités de l'organe de canal de guidage interne et un élément de retenue d'endoprothèse proximal (93).

13. Joint interne selon la revendication 11, dans lequel l'organe de canal de guidage interne (70) comprend en outre un biseau (76) entre la première et la deuxième extrémité (78, 77) des extrémités de l'organe de canal de guidage interne, le biseau (76) ayant un deuxième diamètre extérieur distal (76') inférieur audit diamètre extérieur (75) de la deuxième extrémité.

14. Système d'introduction de dispositif médical destiné à déployer une prothèse implantable au niveau d'un emplacement sélectionné à l'intérieur du corps d'un patient, le système d'introduction comprenant le joint interne selon l'une quelconque des revendications précédentes, ledit dispositif comprenant en outre :
une plate-forme d'endoprothèse (91) disposée circonférentiellement autour de l'organe de canal de guidage interne (70) entre la première et la deuxième extrémité (78, 77) ;
une pointe atraumatique (170) accouplée fonctionnellement à la première extrémité (78) de l'organe de canal de guidage interne, la pointe atraumatique comprenant un élément de retenue d'endoprothèse distal (93') ; et
une bague de retenue disposée autour de, et accouplée fonctionnellement à, l'organe de canal de guidage interne (70) entre la première et la deuxième extrémité (78, 77) de l'organe de canal de guidage interne, la bague de retenue comprenant un élément de retenue d'endoprothèse proximal (93).

15. Dispositif selon la revendication 14, dans lequel le corps de fixation (150) et l'extrémité d'accouplement distale (48) de l'organe de compression interne (41) sont accouplés fonctionnellement par une liaison choisie parmi le groupe constitué d'une soudure, d'un brasage, d'un collage, d'une fusion et de combinaisons de ceux-ci.

16. Dispositif selon la revendication 15, comprenant en outre un tubage thermorétractable (165) disposé autour de la liaison et d'une portion du corps de fixation (150).

17. Dispositif selon la revendication 14, dans lequel la pointe atraumatique (170) comprend en outre une première extrémité distale (178) avec un orifice d'entrée (172) et une deuxième extrémité proximale (177) avec un orifice de sortie (173) et définissant un canal de guidage (171), la deuxième extrémité (177) de la pointe atraumatique comprenant ledit élément de retenue d'endoprothèse distal (93') et étant accouplée fonctionnellement à la première extrémité (78) de l'organe de canal de guidage interne de telle sorte que le canal de guidage (171) de la pointe atraumatique soit en communication fluidique avec le canal de guidage (71) de l'organe de canal de guidage interne (70).

18. Dispositif selon la revendication 14, comprenant en outre un système d'introduction de cathéter à insertion rapide, le système comprenant une gaine externe allongée (50) ayant une extrémité proximale (57) et une extrémité distale (58) définissant un passage (59) entre elles, lequel reçoit par coulissement une portion de l'organe de compression interne (41), l'extrémité distale (58) de la gaine externe étant accouplée fonctionnellement à un organe de canal de guidage externe (80) ayant des première et deuxième extrémités (88, 87) définissant un canal de guidage (81) qui reçoit par coulissement l'organe de canal de guidage interne (70), une ouverture de position de siège (65) au niveau de, ou à proximité de, la deuxième extrémité (87) de l'organe de canal de guidage externe et en communication fluidique avec les deux canaux de guidage (71, 81), et une poignée (30) accouplée fonctionnellement à l'extrémité proximale (57) de la gaine externe et pouvant coulisser axialement par rapport à un stylet (20) accouplé fonctionnellement à l'extrémité proximale (40) de l'organe de compression interne et destinée à rester à l'extérieur dudit patient.

19. Procédé de fabrication d'un joint interne pour l'utilisation dans un système d'introduction de dispositif médical destiné à déployer une prothèse implantable au niveau d'un emplacement sélectionné à l'intérieur du corps d'un patient, comprenant les étapes suivantes :
fournir un organe de canal de guidage interne (70) ayant une première extrémité distale (78) avec un orifice d'entrée (72) et une deuxième extrémité proximale (77) avec un orifice de sortie (73), les orifices d'entrée et de sortie (72, 73) définissant un canal de guidage (71) entre eux, la deuxième extrémité (77) comprenant un diamètre extérieur (75) et une surface externe (102) ;
fournir un corps de fixation (150) définissant une cavité de réception (159) qui est dimensionnée pour recevoir le diamètre extérieur (75) de la deuxième extrémité de l'organe de canal de guidage interne, la cavité de réception (159) ayant au moins une surface d'engagement interne (160), et
le corps de fixation (150) comprenant une portion courbe et une surface d'engagement externe (164) ;
fournir un organe de compression interne allongé (41) ayant une extrémité proximale (40) et une extrémité d'accouplement distale (48) ;
former l'extrémité d'accouplement distal (48) de l'organe de compression interne avec une forme comprenant une surface d'engagement (48') et une configuration profilée (48") complémentaire la surface d'engagement externe (164) du corps de fixation ;
disposer la surface d'engagement externe (164) du corps de fixation en relation d'aboutement avec la surface d'engagement (48') de l'extrémité d'accouplement distal ;
insérer la deuxième extrémité (77) de l'organe de canal de guidage interne à l'intérieur de la cavité de réception (159) du corps de fixation ;
accoupler fonctionnellement la surface d'engagement interne (160) de la cavité de réception et la surface externe (102) de la deuxième extrémité de l'organe de canal de guidage interne.

20. Procédé selon la revendication 19, comprenant en outre l'étape consistant à souder le corps de fixation (150) et l'extrémité d'accouplement distale (48).

21. Procédé selon la revendication 19, comprenant en outre l'étape consistant à braser le corps de fixation (150) et l'extrémité d'accouplement distale (48).

22. Procédé selon la revendication 19, comprenant en outre l'étape consistant à fusionner la surface d'engagement externe (164) du corps de fixation et la surface d'engagement (48') de l'extrémité d'accouplement distale.

23. Procédé selon la revendication 19, dans lequel l'accouplement fonctionnel de la surface d'engagement interne (160) de la cavité de réception et de la surface externe (102) de la deuxième extrémité de l'organe de canal de guidage interne comprend l'étape consistant à former une liaison choisie parmi le groupe constitué d'un sertissage, d'un ajustement par friction, d'un ajustement par pressage, d'une cale, d'une colle, d'un adhésif, d'une résine, d'une soudure, de matériaux de liaison chimique et de combinaisons de ceux-ci.

24. Procédé selon la revendication 19, dans lequel l'étape de formation de la configuration profilée (48") comprend l'étape consistant à rouler ou forger l'extrémité d'accouplement distale (48) de l'organe de compression interne d'une configuration ronde à une configuration cannelée complémentaire à la surface d'engagement externe (164) du corps de fixation.

25. Procédé selon la revendication 19, dans lequel l'étape de formation de la configuration profilée (48") comprend l'étape consistant à rouler ou forger l'extrémité d'accouplement distale (48) de l'organe de compression interne d'une configuration ronde à une configuration plate complémentaire à la surface d'engagement externe (164) du corps de fixation.

26. Procédé selon la revendication 23, dans lequel la formation de la configuration profilée (48") comprend l'étape consistant à forger l'extrémité d'accouplement distale (48) de l'organe de compression interne d'une configuration ronde à une configuration plate complémentaire à la surface d'engagement externe (164) du corps de fixation.
